# EUROPEAN PATENT APPLICATION

(11) **EP 3 715 845 A1**
(43) Date of publication of application: **30.09.2020**
(21) Application number: 19305413.7
(22) Date of filing: 29.03.2019
(51) Int. Cl.: G01N 33/564

(54) **NEW MARKERS FOR DISCRIMINATING JUVENILE IDIOPATHIC ARTHRITIS (JIA) AND SEPTIC ARTHRITIS (SA)**

(71) Applicant: UNIVERSITE DE MONTPELLIER, 34090 Montpellier (FR); Centre Hospitalier Universitaire de Montpellier, 34000 Montpellier (FR); INSTITUT NATIONAL DE LA SANTE ET DE LA RECHERCHE MEDICALE (INSERM), 75013 Paris (FR)
(72) Inventor: LOUIS-PLENCE, Pascale, 34270 Saint Mathieu de Tréviers (FR); CREN, Mailys, 34090 Montpellier (FR); JEZIORSKI, Eric, 34090 Montpellier (FR)
(74) Representative: Regimbeau

(57) **Abstract**

The invention relates to a combination which comprises:
a) at least one compound for detecting a cell surface protein selected in the group consisting of CD14, CD45, CD11c, CD4, HLA-DR, CD1c, CD3, CD19, CD20, and CD56 and mixtures thereof, and optionally CD16, CD86 and/or CD11b, and
b) at least one compound selected in the group consisting of (i) a compound for detecting CD141⁺ Conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and
c) preferably additionally a compound for detecting PDL2 on subsets of dendritic cells selected in the group consisting of CD141⁺cDC, CD123⁺pDCs, CD303⁺ pDCs cells, and mixtures thereof and/or CD1c⁺ cDC cells.

The invention also relates to kits and uses of such compounds in methods for discriminating juvenile idiopathic arthritis (JIA) and septic arthritis (SA) in a subject affected by juvenile arthritis form.

## Description

### FIELD OF THE INVENTION

The present invention relates to the identification of specific markers of subsets of dendritic cells and their use in *in vitro* methods of diagnosing and/or monitoring of juvenile idiopathic arthritis (JIA) or respectively septic arthritis (SA) in a subject affected by juvenile arthritis form. The invention permits to discriminate rapidly between these both arthritis for confirming the diagnosis and defining the adapted treatment.

### BACKGROUND OF THE INVENTION

The two most frequent forms of arthritis among children below the age of 16 years are septic arthritis (SA) and juvenile idiopathic arthritis (JIA) (Aupiais C et al. Arch Dis Child. 2015;100(8):742-747). In most cases, SA results of hematogenous seeding of the vascular synovial membrane due to a bacteremic episode. The main pathogen responsible for SA in childhood is *Staphylococcus aureus,* the second one being *Kingella kingae.* Importantly, in most of cases the infectious agent remains unknown and SA is an orthopedic emergency as delay in diagnosis and treatment may result in irreversible damage to the joint. JIA represents the most common chronic rheumatic disorder in childhood characterized by an articular inflammation that persists for at least 6 weeks. It is a heterogeneous group of immune disorders composed of seven subtypes depending on the number of affected joints, serological features as well as systemic symptoms, the most common type being the oligoarticular JIA. Although the exact etiology of JIA remains unknown, several genetic and environmental factors have been associated with this disorder.

Despite distinct etiology, it remains difficult to distinguish these two entities of arthritis in childhood since they present similar signs and symptoms. Defining an early differential diagnosis is however critical as treatments for SA and JIA are totally distinct. For SA they include an emergent surgical joint drainage and intravenous antibiotics to avoid infectious complications, while JIA requires referral to a rheumatologist who may treat with non-steroidal anti-inflammatory (NSAIDs), intra-articular glucocorticoid injections, disease-modifying anti rheumatic drugs (DMARDS) and/or biologics. However, before the administration of any treatments, joint aspiration is appropriate for every child who presents an initial episode of arthritis lasting less than 6 weeks, especially in case of monoarthritis to avoid SA.

Although some biological parameters including synovial leukocytes count and platelet numbers differed significantly between SA and JIA patients, there is a large overlap zone and none are sufficient to discriminate between JIA and SA. Furthermore, until this day, for diagnosing SA, a culture of synovial fluid is made to obtain a bacterial proliferation and identification; a PCR, that could be specific or universal, may be performed to amplify the bacterial DNA, but in about 50% of cases, septic arthritis (SA) is not confirmed by culture nor by PCR.

So there is still a need to identify specific and reliable markers to distinguish these two juvenile arthritic forms.

The present invention results from an in-depth comparison of various immune cell subsets of both juvenile arthritic forms in two different body fluids (peripheral blood PB and synovial fluid SF), which permits to identify specific and reliable biomarkers able to discriminate between SA and JIA arthropathies. The rapid and reliable diagnosis of SA or JIA thus allows their fast handling for treatment. This would allow a saving of antibiotherapy, avoid diagnostic wanderings, and an earlier start of anti-inflammatory treatment, specific to juvenile arthritis.

### SUMMARY OF THE INVENTION

A first object of the present invention is a combination which comprises:
a) at least one compound for detecting a cell surface protein selected in the group consisting of CD14, CD45, CD11c, CD4, HLA-DR, CD1c, CD3, CD19, CD20, and CD56 and mixtures thereof, and optionally CD16, CD86 and/or CD11b, and
b) at least one compound selected in the group consisting of (i) a compound for detecting CD141⁺ Conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and
c) preferably additionally a compound for detecting PDL2 on subsets of dendritic cells selected in the group consisting of CD141⁺cDC, CD123⁺pDCs, CD303⁺ pDCs cells and mixtures thereof, and/or CD1c⁺ cDC cells.

Another subject-matter of the invention is a kit comprising at least a combination as defined above according to the invention.

The invention also relates to a use of a compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and preferably additionally a compound for detecting PDL2 on said subsets of dendritic cells and/or on CD1c⁺ cDC cells, or use of a combination of compounds as disclosed above according to the invention or a kit as defined in the invention in an *in vitro* method of diagnosing juvenile inflammatory arthritis (JIA) or septic arthritis (SA) and/or monitoring the disease progression of JIA or SA and/or assessing the efficacy of a therapy in a subject affected by JIA or SA.

In a particular embodiment, the invention also concerns a use of a compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and preferably additionally a compound for detecting PDL2 on said subsets of dendritic cells and/or on CD1c⁺ cDC cells, or use of a combination of compounds as disclosed above according to the invention or a kit as defined in the invention for determining, in a subject affected by a juvenile arthritic form, if the juvenile arthritic form is a juvenile inflammatory arthritis (JIA) or is a septic arthritis (SA).

The present invention also relates to an *in vitro* method of quantifying (i) at least one subset of dendritic cells selected in the group consisting of CD141⁺ cDC cells, CD123⁺ pDC cells, CD303⁺ pDC cells, and mixtures thereof, and preferably additionally quantifying (ii) expression level of PDL2 on said subset(s) of dendritic cells and mixtures thereof, and/or on CD1c+ cells, in a biological sample of a subject, in particular in a peripheral blood sample or synovial tissue sample of said subject, comprising:
a) contacting the said biological sample with
   a1) a compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and preferably additionally a compound for detecting PDL2 on said subsets of dendritic cells and/or on CD1c⁺ cDC cells, or
   a2) a combination of compounds as defined above according to the invention,
b) quantifying the binding of said compounds to the said biological sample.

Another object of the invention is an *in vitro* method of diagnosing juvenile inflammatory arthritis (JIA) in a subject, said method comprising the steps of:
a) quantifying according to the invention and as disclosed above, in a biological sample of a subject, in particular in a synovial fluid or synovial tissue sample of said subject, at least one subset of dendritic cells selected in the group consisting of CD141⁺ cDC cells, CD123⁺ pDC cells, CD303⁺ pDC cells, and mixtures thereof,
b) comparing said values of subset(s) of dendritic cells of step a) with a reference value,
wherein an increase of the value of said subset(s) of dendritic cells in comparison to the reference value is indicative of a subject affected by juvenile inflammatory arthritis (JIA).

The expression 'comparing said values of subset(s) of dendritic cells' according to the invention means that the comparison is made between values obtained for comparative 'samples', meaning same type of subset of dendritic cells. For example, if the value in step a) is obtained on CD141⁺ cDC cells alone, then comparison in step b) is made with reference value obtained also on CD141⁺ cDC cells. If the value in step a) is obtained on CD141⁺ cDC cells and CD123⁺ pDC cells, then comparison in step b) is made with reference value obtained also on CD141⁺ cDC cells and CD123⁺ pDC cells.
The 'reference value' is further defined in the description.

The invention also concerns an *in vitro* method of diagnosing septic arthritis (SA) in a subject, said method comprising the steps of:
a) quantifying according to the invention and as disclosed above, in a biological sample of a subject, in particular a peripheral blood sample or synovial fluid or synovial tissue sample of said subject, the expression level of PDL2 on at least one subset of dendritic cells selected in the group consisting of CD141⁺cDC cells, CD123⁺pDCs cells, CD303⁺ pDCs cells, and mixtures thereof and/or CD1c⁺ cDC cells,
b) comparing the expression level of PDL2 on said subset(s) of cells of step a) with a reference value,
wherein an increase of the expression level of PDL2 on said subset(s) of cells in comparison to a reference value is indicative of a subject affected by septic arthritis (SA).

Advantageously, another object of the invention concerns an *in vitro* method of discriminating, in a subject affected by an arthritic form, if the arthritic form is a juvenile inflammatory arthritis (JIA) or a septic arthritis (SA), said method comprising the steps of:
a) implementing the *in vitro* method of diagnosing JIA according to the invention, and in case of no increase of the value of subset(s) of dendritic cells selected in the group consisting of CD141⁺ cDC cells, CD123⁺ pDC cells, CD303⁺ pDC cells and mixtures thereof in comparison to the reference value, indicative of a subject unaffected by JIA,
b) implementing the *in vitro* method of diagnosing SA according to the invention to determine if the subject is affected by SA.

The present invention also concerns an *in vitro* method for monitoring the disease progression in a subject affected by JIA comprising the steps of:
a) quantifying according to the invention and at a first point of time, in a biological sample of a subject, in particular in a synovial fluid or synovial tissue sample of said subject, first values of subset(s) of dendritic cells selected in the group consisting of CD141⁺cDC cells, CD123⁺ pDCs cells, CD303⁺ pDCs cells, and mixtures thereof,
b) quantifying according to the invention and at a second point of time, in a biological sample of a subject, in particular in a synovial fluid or synovial tissue sample of said subject, second values of subset(s) of dendritic cells selected in the group consisting of CD141⁺cDC cells, CD123⁺ pDCs cells, CD303⁺ pDCs cells, and mixtures thereof,
c) comparing the first and second values obtained at step a) and step b), and
d) monitoring the disease progression in the subject affected by JIA from the said comparison.

The invention also relates to an *in vitro* method for monitoring the disease progression in a subject affected by SA comprising the steps of:
a) quantifying according to the invention and at a first point of time, in a biological sample of a subject, in particular in a peripheral blood sample or synovial tissue sample of said subject, first values for expression level of PDL2 on subset(s) of dendritic cells selected in the group consisting of CD141⁺cDC cells, CD123⁺ pDCs cells, CD303⁺ pDCs cells, and mixtures thereof and/or CD1c⁺ cDC cells,
b) quantifying according to the invention and at a second point of time, in a biological sample of the same subject, in particular in a peripheral blood sample or synovial tissue sample of said subject, second values for expression level of PDL2 on subset(s) of dendritic cells selected in the group consisting of CD141⁺cDC cells, CD123⁺ pDCs cells, CD303⁺ pDCs cells and mixtures thereof, and/or CD1c⁺ cDC cells,
c) comparing the first and second values obtained at step a) and step b), and
d) monitoring the disease progression in the subject affected by SA from the said comparison.

The invention also relates to an *in vitro* method for assessing the efficacy of a therapy in a subject affected by JIA, comprising the steps of:
a) quantifying according to the invention as disclosed above, in a biological sample of a subject before said treatment, in particular in a synovial fluid or synovial tissue sample of said subject, first values of subset(s) of dendritic cells selected in the group consisting of CD141⁺cDC cells, CD123⁺ pDCs cells, CD303⁺ pDCs cells, and mixtures thereof,
b) quantifying according to the invention as disclosed above, in a biological sample of the same subject during or after said treatment, in particular in a synovial fluid or synovial tissue sample of said subject, first values of subset(s) of dendritic cells selected in the group consisting of CD141⁺cDC cells, CD123⁺ pDCs cells, CD303⁺ pDCs cells, and mixtures thereof,
c) assessing the efficacy of the therapy based on the comparison of the second values obtained in step b) with the first values obtained at step a).

Another object of the invention is an *in vitro* method for assessing the efficacy of a therapy in a subject affected by SA, comprising the steps of:
a) quantifying according to the invention as disclosed above, in a biological sample of a subject before said treatment, in particular in a peripheral blood sample or synovial tissue sample of said subject, first values for expression level of PDL2 on subset(s) of dendritic cells selected in the group consisting of CD141⁺cDC cells, CD123⁺ pDCs cells, CD303⁺ pDCs cells, and mixtures thereof and/or CD1c⁺ cDC cells,
b) quantifying according to the invention as disclosed above, in a biological sample of the same subject during or after said treatment, in particular in a peripheral blood sample or synovial tissue sample of said subject, first values for expression level of PDL2 on subset(s) of dendritic cells selected in the group consisting of CD141⁺cDC cells, CD123⁺ pDCs, cells, CD303⁺ pDCs cells and mixtures thereof and/or CD1c⁺ cDC cells,
c) assessing the efficacy of the therapy based on the comparison of the second values obtained in step b) with the first values obtained at step a).

The present invention also relates to a use of a cell surface protein selected in the group consisting of CD141, CD123, CD303, and mixtures thereof as specific markers of dendritic cells for diagnosing JIA and/or monitoring the disease progression JIA and/or assessing the efficacy of a therapy in a subject affected by juvenile inflammatory arthritis (JIA) from biological sample of a subject, in particular a synovial fluid or synovial tissue sample of a subject.

The present invention also concerns use of PDL2 as a specific marker of dendritic cells for diagnosing SA and/or monitoring the disease progression SA and/or assessing the efficacy of a therapy in a subject affected by septic arthritis (SA) from a peripheral blood sample or a synovial fluid or synovial tissue sample of a subject.

The invention mainly refers to combinations of compounds for detecting specific markers of dendritic cells, and uses thereof for diagnosing and/or discriminating between JIA and SA. But said compounds for detecting specific markers of dendritic cells may also be combined to other specific markers for monocytes, such as the ones illustrated in the examples.

### BRIEF DESCRIPTION OF THE FIGURES

Figure 1A: Heatmap of supervised hierarchical clustering of cell counts obtained from cytometric analyses with a false discovery rate (FDR) cutoff of 5%.
Figure 1B: Numbers of classical (CD14⁺⁺CD16⁻) monocyte subsets in PB of SA subjects and JIA subjects;
Figure 1C: Numbers of the classical (CD14⁺⁺CD16⁻), intermediate (CD14⁺⁺CD16⁺) and non-classical (CD14⁺CD16⁺⁺) monocyte subsets in SF of SA subjects and JIA subjects;
Figure 1D: Numbers of CD141⁺ cDCs and CD123⁺ pDCs cells in SF of SA subjects and JIA subjects;
Figure 2A: Expression of CD64 (Mean Fluorescence Intensity, MFI) on the three monocyte subsets in PB and SF of SA subjects and JIA subjects;
Figure 2B: Expression of SLAN (MFI) on the three monocyte subsets in PB and SF of SA subjects and JIA subjects;
Figure 2C: Expression of HLA-DR (MFI) on the three monocyte subsets in PB and SF of SA subjects and JIA subjects;
Figure 2D: Expression of HLA-DR (MFI) on CD1c⁺ cDC, CD141⁺ cDCs, CD123⁺ pDC and CD14⁺ InflDCs in PB and SF of SA subjects and JIA subjects;
Figure 2E: Expression of CD86 (MFI) on CD1c⁺ cDC, CD141⁺ cDCs and CD14⁺ InflDCs in PB and SF of SA subjects and JIA subjects;
Figure 2F: Expression of PDL2 (MFI) on all four DC subsets in PB and SF of SA subjects and JIA subjects;
Figure 3: Monitoring of Blood pDC numbers and disease duration.

### DETAILED DESCRIPTION OF THE INVENTION

Several lines of evidence suggest that innate immunity plays a pivotal role in JIA and SA. Innate immune cells indeed represent the first line of defense against pathogens and have a critical role in initiating and orchestrating the inflammation through the production of pro-inflammatory cytokines and chemokines. The main immune cells involved in innate immune response are dendritic cells (DCs) or monocytes.

DCs are key players in initiating immune response and maintaining tolerance. They sample antigens from the environment and present them as peptide-MHC complexes to effector T cells in lymphoid organs. In peripheral blood, DCs comprise a heterogeneous population of cells that is divided into three main subsets, two myeloid or conventional DCs (cDC) referred as BDCA1/CD1c⁺ cDCs and BDCA3/CD141⁺ cDCs, and plasmacytoid DCs (pDC) characterized by the expression of CD123 (IL-3R). These three DC populations are found in all lymphoid organs as well, and represent resident DCs. DC subsets have been characterized in immune disorders, including JIA.

The present inventors have surprisingly found that JIA subjects display a higher proportion of a specific class of dendritic cells. More specifically, the present inventors have found that the subsets of conventional dendritic cells expressing CD141 (CD141⁺ cDC) and plasmacytoid dendritic cells expressing CD123 (CD123⁺ pDC) or CD303 (CD303⁺ pDC) are hyper-represented in the synovial fluid of JIA subjects, in comparison to SA subjects. Therefore, the proportion of CD141⁺ cDC and/or CD123⁺ pDC and/or CD303⁺ pDC in a synovial fluid sample of a subject can be used as a positive diagnosis criterion for JIA.

They also demonstrated that the expression level of PDL2 on CD141⁺ cDC and CD123⁺ pDC from peripheral blood sample (PB) or synovial fluid sample (SF) of a subject suffering from SA is significantly increased in comparison with JIA patients. PDL-2 expression was also increased on CD1c+ cDC and CD14+lnfDC in PB and SF of SA compare with JIA patients.

The invention thus enables the skilled person to identify those subjects who are affected by JIA or SA by simply quantifying the CD141⁺ cDC and/or CD123⁺ pDC subsets of cells from synovial fluid sample from said subjects and/or quantifying the PDL2 expression level on CD1c+ DC, and/or CD141⁺ cDC, and/or CD123⁺ pDC, and/or CD303⁺ pDC, and/or CD14⁺InfDCs of a peripheral blood sample or synovial fluid sample from said subjects. These parameters can be determined in less than 48 hours. Thus, the *in vitro* method of the invention is particularly advantageous because it generates a diagnosis in a very short time and with a very high degree of confidence, whereas the current methods are both time-consuming and prone to mis-identification.

### Definitions

A "subject" which may be subjected to the *in vitro* methods described herein may be any of mammalian animals including human. More preferably, the subject of the invention is human subject; a human subject can be known as a patient. In one embodiment, "subject" or "subject affected by an arthritis form" refers to a mammal, preferably a human that is affected by JIA or SA or has been diagnosed with JIA or SA. According to the present invention, the "subject" is selected in the group consisting of children below the age of 18, in particular below the age of 16, and more particularly from the age of 6 months to 16 years old, affected or suspected to be affected by an arthritis, in particular selected in the group consisting of JIA or SA.

A "control subject" that may be used in some embodiment of the *in vitro* method of the present invention, in comparison to "subject", refers to a mammal, preferably a human, which is not affected by JIA or SA, and is not suspected of being diagnosed with JIA or SA.

As used herein, the term "biological sample" or "sample" refers to a whole organism or a subset of its tissues, cells or components parts. In a particular embodiment, the "biological sample" refers to a sample comprising cells, preferably dendritic cells. In another particular embodiment, the "biological sample" refers to a sample comprising synovial tissue, in particular synovial tissue from a biopsy. "Biological sample" further refers to a homogenate, lysate or extract prepared from a whole organism or a subset of its tissues, cells or component parts, or a fraction or portion thereof. The biological sample to be measured in the in *vitro* methods of the present invention is not particularly limited, as far as it can be collected from a mammal, preferably from a human; examples include humoral samples such as blood, bone marrow fluid, and lymph fluid, and solid samples such as lymph nodes, blood vessels, bone marrow, brain, spleen, and skin. Preferably, a "biological sample" according to the invention is peripheral blood or synovial fluid or synovial tissue containing dendritic cells.

It is particularly advantageous to use peripheral blood (PB), synovial fluid (SF) or synovial tissue from a biopsy as a biological sample for the *in vitro* method of the invention. Indeed, such a blood sample or synovial fluid or synovial tissue sample may be obtained by a completely harmless blood collection or synovial fluid or synovial tissue (for example from a biopsy) from the subject and thus allows for a non-invasive diagnosis of SA or JIA.

In a first embodiment, the biological sample is peripheral blood (PB) or synovial fluid (SF) or synovial tissue sample for *in vitro* methods of the invention related to SA.

In another embodiment, the biological sample is synovial fluid or synovial tissue sample, preferably synovial fluid (SF) sample for the *in vitro* methods of the invention related to JIA.

For example, the required volume of the biological sample used in the *in vitro* methods of the invention is a volume containing at least 0.5 million of dendritic cells, in particular at least 0.6 million of dendritic cells, and more particular at least 0.7 million of dendritic cells. It will generally represent a volume of at least 300µl of blood sample and a volume of at least 100µl of synovial fluid.

It is preferable that the blood sample or synovial fluid sample used in the method of the invention be fresh. By "a fresh blood or synovial fluid sample", it is herein referred to a sample of blood or synovial fluid which has been drawn within the previous 6 hours, preferably 4 hours, 3 hours, 2 hours, 1 hour, 30 minutes, or 15 minutes. Preferentially, the fresh blood or synovial fluid sample of the invention will be kept at 20°C until used.

According to the present invention, a cell "expresses CD141" (CD141 is used as an example but the definition applies to other CD markers used in the present invention) if CD141 is present at a significant level on its surface (such a cell being also defined as a "CD141⁺ cell"). In particular, a cell expresses CD141 if the signal associated to surface CD141 staining (e.g. obtained with an antibody anti-CD141 coupled to a fluorescent marker) which is measured for said cell is superior to the signal corresponding to the staining of one cell being known as not expressing CD141.

In a preferred embodiment, CD141⁺ cells are such that the ratio between the surface CD141- associated signal measured for said cells and the surface CD141- associated signal measured for cells being known as expressing CD141 is positive (i.e., above 0).

By 'monitoring the progression of the disease' according to the invention, it means quantifying, in a same subject, at different points of times, the specific markers of the diseases (CD141 and/or CD123 and/or CD303 for JIA; and PDL2 for SA) and determine, from the comparison between the different points of time, if the subject is in a better health (the disease regresses) or if he is in a worst health (the disease progresses). An increase of the quantified values of the markers is indicative of a progression of the disease and a decrease of the quantified values is indicative of a regression of the disease.

### Combination of compounds

A first object of the present invention is a combination which comprises:
a) at least one compound for detecting a cell surface protein selected in the group consisting of CD14, CD45, CD11c, CD4, HLA-DR, CD1c, CD3, CD19, CD20, and CD56 and mixtures thereof, and optionally CD16, CD86 and/or CD11b, and
b) at least one compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and
c) preferably additionally a compound for detecting PDL2 on subsets of dendritic cells selected in the group consisting of CD141⁺cDC, CD123⁺pDCs, CD303⁺ pDCs cells, and mixtures thereof and/or on CD1c⁺ cDC cells.

By 'combination' according to the invention, it means a 'combination of products', ie a combination of compounds comprising at least one compound for detecting the specific markers of dendritic cells in interest for the invention (compound selected in the group consisting of CD141, CD123, CD303, and mixtures thereof, and preferably additionally PDL2) and at least one compound for detecting other markers of cells to isolate/eliminate. The said compounds are used in combination, simultaneously and/or separately (for example sequentially) in the different steps of the in *vitro* methods of the invention.

In particular, the combination of the invention comprises a) at least a compound for detecting a cell surface protein specific for cells to isolate/eliminate, b) at least one compound for detecting a cell surface protein specific of the dendritic cells to be selected (CD141⁺ cDC cells, CD123⁺ pDC cells, CD303⁺ pDC cells, and mixtures thereof) and c) preferably additionally a compound for detecting PDL2 on said subset(s) of dendritic cells and/or on CD1c⁺ cDC cells.
Each of the compounds b) may be used alone or in combination with another compound b) ('mixture thereof).
In a first embodiment, a combination of the invention comprises:
a) at least one compound for detecting a cell surface protein selected in the group consisting of CD14, CD45, CD11c, CD4, HLA-DR, CD1c, CD3, CD19, CD20, and CD56 and mixtures thereof, and optionally CD16, CD86 and/or CD11b, and
b) a compound for detecting CD141⁺ conventional dendritic cells (cDC), and
c) preferably additionally a compound for detecting PDL2 on subsets of dendritic cells selected in the group consisting of CD141⁺cDC, CD123⁺pDCs, CD303⁺ pDCs cells, and mixtures thereof and/or on CD1c⁺ cDC cells.

In another embodiment, a combination of the invention comprises :
a) at least one compound for detecting a cell surface protein selected in the group consisting of CD14, CD45, CD11c, CD4, HLA-DR, CD1c, CD3, CD19, CD20, and CD56 and mixtures thereof, and optionally CD16, CD86 and/or CD11b, and
b) a compound for detecting CD123⁺ plasmacytoid dendritic cells (pDC) or a compound for detecting CD303⁺ plasmacytoid dendritic cells (pDC),
c) preferably additionally a compound for detecting PDL2 on subsets of dendritic cells selected in the group consisting of CD141⁺cDC, CD123⁺pDCs, CD303⁺ pDCs cells, and mixtures thereof and/or on CD1c⁺ cDC cells.

In another embodiment, a combination of the invention comprises :
a) at least one compound for detecting a cell surface protein selected in the group consisting of CD14, CD45, CD11c, CD4, HLA-DR, CD1c, CD3, CD19, CD20, and CD56 and mixtures thereof, and optionally CD16, CD86 and/or CD11b, and
b) a compound for detecting CD141⁺ conventional dendritic cells (cDC) and a compound for detecting CD123⁺ plasmacytoid dendritic cells (pDC),
c) preferably additionally a compound for detecting PDL2 on subsets of dendritic cells selected in the group consisting of CD141⁺cDC, CD123⁺pDCs, CD303⁺ pDCs cells, and mixtures thereof and/or on CD1c⁺ cDC cells.

In another embodiment, a combination of the invention comprises :
a) at least one compound for detecting a cell surface protein selected in the group consisting of CD14, CD45, CD11c, CD4, HLA-DR, CD1c, CD3, CD19, CD20, and CD56 and mixtures thereof, and optionally CD16, CD86 and/or CD11b, and
b) a compound for detecting CD141⁺ conventional dendritic cells (cDC) and a compound for detecting CD303⁺ plasmacytoid dendritic cells (pDC),
c) preferably additionally a compound for detecting PDL2 on subsets of dendritic cells selected in the group consisting of CD141⁺cDC, CD123⁺pDCs, CD303⁺ pDCs cells, and mixtures thereof and/or on CD1c⁺ cDC cells.

By 'a compound for detecting X' according to the invention, it means a compound able to bind to 'X'. 'X' may be a peptide or a protein (e.g a cell surface protein or antigen expressed at the surface of dendritic cells), or a nucleic acid sequence (ex: mRNA). In a particular embodiment, 'X' is a cell surface protein or antigen expressed at the surface of dendritic cells, ie a cluster of differentiation (CD) of dendritic cells (DC), such as CD141, CD123, CD303 and PDL2.

'Cell surface proteins' are commonly known as proteins that are embedded in or span the layer of cell membranes of more complex organisms. These proteins are integral to the way in which a cell interacts with the environment around it, including other cells. Some of these proteins, especially ones that are exposed to the external side of the membrane, are called glycoproteins because they have carbohydrates attached to their outer surfaces.
'Cluster of differentiation' (abbreviated as CD) is commonly known as a protocol used for the identification and investigation of cell surface molecules providing targets for immunophenotyping of cells. In terms of physiology, CD molecules can act in numerous ways, often acting as receptors or ligands important to the cell. A signal cascade is usually initiated, altering the behavior of the cell.

In the present disclosure, the terms 'cell surface protein', 'cluster of differenciation' (CD), or 'marker' will be used interchangeably.

As disclosed above DCs comprise a heterogeneous population of cells that is divided into three main subsets, two myeloid or conventional DCs (cDC) referred as BDCA1/CD1c⁺ cDCs and BDCA3/CD141⁺ cDCs, and plasmacytoid DCs (pDC) characterized by the expression of CD123 (IL-3R).
'CD1c' is a member of the CD1 family of transmembrane glycoproteins, which are structurally related to the major histocompatibility complex (MHC) proteins and form heterodimers with beta-2-microglobulin. The CD1 proteins mediate the presentation of primarily lipid and glycolipid antigens of self or microbial origin to T cells. The human genome contains five CD1 family genes organized in a cluster on chromosome 1. The CD1 family members are thought to differ in their cellular localization and specificity for particular lipid ligands. The protein encoded by this gene is broadly distributed throughout the endocytic system via a tyrosine-based motif in the cytoplasmic tail. Alternatively spliced transcript variants of this gene have been observed, but their full-length nature is not known.
The protein sequence of human CD1c is represented by the reference sequence NCBI: NP_001756.2 (T-cell surface glycoprotein CD1c precursor) or by the reference sequence NCBI: XP_005245636.1 (T-cell surface glycoprotein CD1c isoform X1), and is encoded by the human CD1c gene (NCBI reference: Gen ID: 911), whose sequence corresponds to the reference NCBI: NM_001765.3 (mRNA).

'CD141' also named thrombomodulin (THBD) is an endothelial-specific type I membrane receptor that binds thrombin. This binding results in the activation of protein C, which degrades clotting factors Va and Villa and reduces the amount of thrombin generated. Mutations in this gene are a cause of thromboembolic disease, also known as inherited thrombophilia.
The protein sequence of human CD141 is represented by the reference sequence NCBI: NP_000352.1 (thrombomodulin precursor), and is encoded by the human CD141 gene (NCBI reference: Gene ID: 7056), whose sequence corresponds to the reference NCBI: NM_000361.2 (mRNA).

'CD123' also named interleukin 3 receptor subunit alpha is an interleukin 3 specific subunit of a heterodimeric cytokine receptor. The receptor is comprised of a ligand specific alpha subunit and a signal transducing beta subunit shared by the receptors for interleukin 3 (IL3), colony stimulating factor 2 (CSF2/GM-CSF), and interleukin 5 (IL5). The binding of this protein to IL3 depends on the beta subunit. The beta subunit is activated by the ligand binding, and is required for the biological activities of IL3. This gene and the gene encoding the colony stimulating factor 2 receptor alpha chain (CSF2RA) form a cytokine receptor gene cluster in a X-Y pseudoautosomal region on chromosomes X or Y. Alternatively spliced transcript variants encoding distinct isoforms have been found.
The protein sequence of human CD123 (interleukin 3 receptor subunit alpha) is represented by the following references sequences NCBI related to its isoforms:
- interleukin 3 receptor subunit alpha isoform 1 precursor: NP_002174.1;
- interleukin 3 receptor subunit alpha isoform 2 precursor: NP_001254642.1;
- interleukin 3 receptor subunit alpha isoform X1: XP_005274837.1 or XP_005274488.1; and
- interleukin 3 receptor subunit alpha isoform X2: XP_016885532.1, or XP_016884980.1, or XP_005274838.1, or XP_005274489.1;
and is encoded by the human CD123 gene (NCBI reference: Gen ID: 3563), whose sequence corresponds to the reference NCBI: NM_002183.3 (transcript variant 1) or NCBI: NM_001267713.1 (transcript variant 2).

'CD303' also known as C-type lectin domain family 4 member C, encodes a member of the C-type lectin/C-type lectin-like domain (CTL/CTLD) superfamily. Members of this family share a common protein fold and have diverse functions, such as cell adhesion, cell-cell signalling, glycoprotein turnover, and roles in inflammation and immune response.

The encoded type 2 transmembrane protein may play a role in dendritic cell function. Two transcript variants encoding distinct isoforms have been identified for this gene.
The protein sequence of human CD303 is represented by the following references sequences NCBI related to its isoforms:
- C-type lectin domain family 4 member C isoform 1 : NP_569708.1;
- C-type lectin domain family 4 member C isoform 2 : NP_987099.1;
- C-type lectin domain family 4 member C isoform X1 : XP_024304641.1 or XP_024304640.1;
- C-type lectin domain family 4 member C isoform X2 : XP_016874429.1
and is encoded by the human CD303 gene (NCBI reference: Gen ID: 170482), whose sequence corresponds to the reference NCBI: NM_130441.2 (transcript variant 1) or NM_203503.1 (transcript variant 2).

'PDL2' is also named programmed cell death 1 ligand 2 (PDCD1LG2).
The protein sequence of human PDL2 is represented by the reference sequence NCBI: NP_079515.2 (isoform 1) or XP_005251657.1 (isoform X1), and is encoded by the human PDL2 gene (NCBI reference: Gen ID: 80380), whose sequence corresponds to the reference NCBI: NM_025239.3 (mRNA).

Numerous antibodies against respectively human CD1c, CD141, CD123, CD303 and PDL2 are commercially available, for example purchased by BD Biosciences or by Miltenyi Biotec, Germany.

In a particular combination of the invention as defined above:
a) the compound(s) for detecting a cell surface protein selected in the group consisting of CD14, CD45, CD11c, CD4, HLA-DR, CD1c, CD3, CD19, CD20, and/or CD56 and mixtures thereof, and optionally CD16, CD86, and/or CD11b, is (are) anti-CD14, anti-CD45, anti-CD11c, anti-CD4, anti-HLA-DR, anti-CD1c, anti-CD3, anti-CD19, anti-CD20, anti-CD56 and mixtures thereof, and optionally anti-CD16, anti-CD86 and/or anti-CD11 b antibodies,
b) the compound (i) for detecting CD141⁺ cDC cells is an anti-CD141 antibody, the compound (ii) for detecting CD123⁺ pDC cells is an anti-CD123 antibody and the compound (iii) for detecting CD303⁺ pDC is an anti-CD303 antibody, and
c) the compound for detecting PDL2 is selected in the group consisting of (x) an anti-PDL2 antibody, (y) a pairs of primers capable of hybridizing at least a part of human PDL2 sequence (Gene: ID: 80380) or (z) a probe capable of hybridizing at least a part of human PDL2 sequence (Gene: ID: 80380).

The compounds, in particular antibodies according to the invention, may be bound or coupled to solid support.
The term 'solid support' as used herein refers to an inert material or molecule to which an antibody may be bound our coupled, either directly or indirectly through a linking group. The solid support is suitable for use in column chromatography or other types of purification. For diagnostic immunoassay, solid supports may be the same as or different from the types of solid supports used for chromatography, with suitable examples including but not limited to beads, plates, slides, or wells formed from materials such as latex or polystyrene. In a particular embodiment, solid support comprises beads.

The term "antibody" as used herein is intended to include monoclonal antibodies, polyclonal antibodies, and chimeric antibodies. Antibody fragments can also be used in the *in vitro* methods of the invention. This term is intended to include Fab, Fab', F(ab')2, scFv, dsFv, ds-scFv, dimers, minibodies, diabodies, and multimers thereof and bispecific antibody fragments. Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, diabodies, bispecific antibody fragments and other fragments can also be synthesized by recombinant techniques. In a particular embodiment, the antibodies are monoclonal antibodies.

The antibodies used in the combinations, kits and *in vitro* methods of the invention can be of different isotypes (namely IgA, IgD, IgE, IgG or IgM).

They may be from recombinant sources and/or produced in transgenic animals. Conventional techniques of molecular biology, microbiology and recombinant DNA techniques are within the skill of the art. Such techniques are explained fully in the literature.

Commercial antibodies recognizing specifically the antigens expressed by dendritic cells can be furthermore used. Some of them are listed in the experimental part below (said list being however not exhaustive nor limitating).

These antibodies can be detected by direct labeling with detectable markers. Alternatively, unlabeled primary antibody can be used in conjunction with a labeled secondary antibody, comprising antisera, polyclonal antisera or a monoclonal antibody specific for the primary antibody. Immunohistochemistry protocols and kits are well known in the art and are commercially available.

In a preferred embodiment of the invention, these antibodies are labelled with a detectable marker, preferably a fluorescent or a luminescent marker. Examples of detectable markers / labels include various enzymes, prosthetic groups, fluorescent materials, luminescent materials, bioluminescent materials, and radioactive materials, preferably fluorescent materials.

Examples of suitable enzymes include horseradish peroxidase, alkaline phosphatase, beta-galactosidase, or acetylcholinesterase examples of suitable prosthetic group complexes include streptavidin/biotin and avidin/biotin, examples of suitable fluorescent materials include umbelliferone, fluorescein, fluorescein isothiocyanate, rhodamine, dichlorot[pi]azinylamine fluorescein, dansyl chloride or phycoerythrin, an example of a luminescent material includes luminol, examples of bioluminescent materials include luciferase, luciferin, and aequorin, and examples of suitable radioactive material include ¹²⁵I, ¹³¹I, ³⁵S or ³H. In a particular and preferred embodiment, the antibodies are labelled with a fluorescent material.

'Primers' and respectively 'probe' of the present invention have common definitions.
The term 'primer' refers to an oligonucleotide, either RNA or DNA, when placed in the proper environment, is able to functionally act as an initiator of template-dependent nucleic acid synthesis.
The term 'probe' is an oligonucleotide, polynucleotide or nucleic acid which is capable of specifically hybridizing to a nucleic acid with sequences complementary to the probe. The person skilled in the art will define specific (y) pair of primers capable of hybridizing at least a part of human PDL2 sequence (Gene: ID: 80380) or (z) probes capable of hybridizing at least a part of human PDL2 sequence (Gene: ID: 80380).
The pair of primers is used, for example in PCR technologies for amplifying a part of human PDL2 sequence (Gene: ID: 80380). The probe is used to hybridize a specific part of human PDL2 sequence (Gene: ID: 80380). Such specific part of human PDL2 nucleic sequence may be part of the coding sequence (CDS). A person having ordinary skill in the art would easily design specific primers to perform either RT-PCR or PCR.

In a particular embodiment, the combination and *in vitro* methods of the invention use commercially available anti-CD141, anti-CD123, anti-CD303 and anti-PDL2 antibodies. Mention may be made to commercially available anti-CD141, anti-CD123, anti-CD303 and anti-PDL2 antibodies sold by BD Bioscience or by Miltenyi Biotec, Germany.

In a preferred embodiment, the combination of the invention comprises at least one additional antibody selected in the group consisting of anti-CD14, anti-CD45, anti-CD11c, anti-CD4, anti-HLA-DR, anti-CD1c, anti-CD3, anti-CD19, anti-CD20, anti-CD56 antibodies and mixtures thereof, and optionally anti-CD16, anti-CD86 and/or anti-CD11 b antibodies. Such additional antibodies may be of interest, in the case of 'flow cytometry' methods to detect and optionally isolate and/or eliminate the different subsets of cells and quantify the subsets of interest (CD141+ cDC, CD123+ pDC or CD303+ pDC).

Advantageously, the said combination comprises at least 13 antibodies comprising a) anti-CD14, anti-CD45, anti-CD11c, anti-CD4, anti-HLA-DR, anti-CD1c, anti-CD3, anti-CD19, anti-CD20, anti-CD56 antibodies, b) anti-CD141 antibody, anti-CD123 or anti-CD303 antibodies, and (c) anti-PDL2 antibody, and optionally anti-CD16, anti-CD86 and/or anti-CD11b antibodies. The use of the combination of said 13 antibodies is advantageous in the context of the invention, in particular for *in vitro* methods of the invention as disclosed hereunder and performed by flow cytometry, as it permits to detect the cells of interest in one step.

In particular, the said antibodies are labelled with a detectable marker, in particular selected in the group consisting of enzymes, prosthetic groups, fluorescent materials, luminescent materials, bioluminescent materials, and radioactive materials, preferably fluorescent materials.

In a particular embodiment, the specific fluorochrome (fluorescent material) is selected in the group consisting of PE, APC, PE-Cy™5, Alexa Fluor® 647, PE-Cy7, PerCP-Cy™5.5, Alexa Fluor® 488, Pacific Blue, FITC, AmCyan, APC-Cy™7, PerCP, BD™APC-H7, BD Horizon™BV786, BD Horizon™BV711, BD Horizon™BV650, BD Horizon™BV605, BD Horizon™BV510, V500, BD Horizon™BV421, V450, PE-Cy™7, BD Horizon™PE-CF594, Alexa Fluor®700, APC/Alexa Fluor®750, BD Horizon™BUV395, BD Horizon™BUV737.

In a particular and preferred embodiment, the present invention uses the following fluorochromes: BD Horizon™BV786, BD Horizon™BV711, BD Horizon™BV650, BD Horizon™BV605, V500, BD Horizon™BV421, Alexa Fluor® 488, PerCP-Cy™5.5, PE-Cy™7, BD Horizon™PE-CF594, APC/ AlexaFluor 750, Alexa Fluor®700, PE and APC.

In particular, the said labelled antibodies are selected in the group consisting of a) anti-CD14- BD Horizon™BV650, anti-CD45-V500, anti-CD11c-BD Horizon™BV421, anti-CD4- Alexa Fluor® 488, anti-HLA-DR-APC/ AlexaFluor 750, anti-CD1c-PE, anti-CD3-Alexa Fluor®700, anti-CD19- Alexa Fluor®700, anti-CD20-Alexa Fluor®700, anti-CD56-Alexa Fluor®700 and mixtures thereof, b) anti-CD141-BD Horizon™BV711, anti-CD123-PerCP-Cy™5.5, anti-CD303-APC and mixtures thereof and c) anti-PDL2- BD Horizon™BV786, and optionally anti-CD16-BD Horizon™PE-CF594, anti-CD86-PE-Cy™7, and/or anti-CD11 b- BD Horizon™BV605 antibodies.

In particular, the said labelled antibodies used according to the present invention and further illustrated in the examples, are listed hereunder : anti-CD14-BD Horizon™BV650 (Ref : 563419, BD Biosciences), anti-CD11 b-BD Horizon™BV605 (Ref: 562721, BD Biosciences), anti-CD45-V500 (Ref 560777, BD Biosciences), anti-CD11c-BD Horizon™BV421 (Ref 562561, BD Biosciences), anti-CD4-Alexa Fluor® 488 (Ref 557695, BD Biosciences), anti-CD86- PE-Cy™7 (Ref 561128, BD Biosciences), anti-CD16-BD Horizon™PE-CF594 (Ref 562293, BD Bosciences), anti-CD1c-PE (Ref 130-090-508, Miltenyi Biotech), anti-HLA-DR-APC/ AlexaFluor 750 (Ref B42021, Beckman Coulter), anti-CD3-Alexa Fluor®700 (Ref 557943, BD Biosciences), anti-CD19-Alexa Fluor®700 (557921, BD Biosciences), anti-CD20-Alexa Fluor®700 (Ref 560631, BD Biosciences), and anti-CD56-Alexa Fluor®700 (Ref 557919, BD Biosciences), anti-CD141-BD Horizon™BV711 (Ref 563155, BD Biosciences), anti-CD123- PerCP-Cy™5.5 (Ref 558714, BD Biosciences) or anti-CD303-APC (Ref 130-113-590, Miltenyi Biotech), and anti-PDL2-BD Horizon™BV786 (Ref: 563843, BD Biosciences).
These commercial references are cited as non-limitative examples of commercial antibodies.

An optimal combination of antibodies according to the invention comprises 13 antibodies with 10 different fluorochromes: a) anti-CD14- BD Horizon™BV650, anti-CD45-V500, anti-CD11c-BD Horizon™BV421, anti-CD4- Alexa Fluor® 488, anti-HLA-DR-APC/ AlexaFluor 750, anti-CD1c-PE, anti-CD3- Alexa Fluor®700, anti-CD19- Alexa Fluor®700, anti-CD20-Alexa Fluor®700, anti-CD56-Alexa Fluor®700, b) anti-CD141-BD Horizon™BV711, anti-CD123- PerCP-Cy™5.5 or anti-CD303-APC, and c) anti-PDL2- BD Horizon™BV786.

Another combination of antibodies according to the invention comprises 15 antibodies (including anti-CD16 and anti-CD86) with 12 different fluorochromes: a) anti-CD14-BD Horizon™BV650, anti-CD45-V500, anti-CD11c-BD Horizon™BV421, anti-CD4-Alexa Fluor® 488, anti-HLA-DR-APC/Alexa Fluor®750, anti-CD1c-PE, anti-CD3-Alexa Fluor®700, anti-CD19-Alexa Fluor®700, anti-CD20-Alexa Fluor®700, anti-CD56-Alexa Fluor®700, anti-CD16-BD Horizon™PE-CF594, anti-CD86-PE-Cy7, b) anti-CD141-BD Horizon™BV711, anti-CD123-PerCP-Cy™5.5 or anti-CD303-APC, and c) anti-PDL2-BD Horizon™BV786.

Another combination of antibodies according to the invention comprises 17 antibodies with 14 different fluorochromes: (a) anti-CD14-BD Horizon™BV650, anti-CD45-V500, anti-CD11c-BD Horizon™BV421, anti-CD4-Alexa Fluor® 488, anti-HLA-DR-APC/ AlexaFluor 750, anti-CD1c-PE, anti-CD3-Alexa Fluor®700, anti-CD19-Alexa Fluor®700, anti-CD20-Alexa Fluor®700, anti-CD56-Alexa Fluor®700, anti-CD16-BD Horizon™PE-CF594, anti-CD86- PE-Cy™7, anti-CD11b- BD Horizon™BV605, anti-CD303-APC, b) anti-CD141-BD Horizon™BV711 anti-CD123-PerCP-Cy™5.5 and c) anti-PDL2-BD Horizon™BV786.

So the combination of antibodies according to the present invention comprises at least 13 antibodies, and optionally at least 14 antibodies (with CD16), 15 antibodies (with CD16 and CD86), 16 antibodies (with CD16, CD303 and CD86) or even 17 antibodies (with CD16, CD303, CD86 and CD11b).

### Kit and uses thereof

Another subject-matter of the invention is a kit comprising at least a combination of compounds as defined above.

In a particular embodiment, the kit is a kit for detecting at least one subset of dendritic cells selected in the group consisting of CD141+ cDC cells, CD123+ pDC cells, CD303+ pDC cells and mixtures thereof, within the biological sample containing other cells. So the kit of the invention comprises a) and at least one antibody selected in the group consisting of anti-CD14, anti-CD45, anti-CD11c, anti-CD4, anti-HLA-DR, anti-CD1c, anti-CD3, anti-CD19, anti-CD20, anti-CD56 and mixtures thereof, b) at least one antibody selected in the group consisting anti-CD141 anti-CD123, anti-CD303 antibodies and mixtures thereof, and optionally anti-CD16, anti-CD86 and/or anti-CD11 b antibodies.

Preferably the kit comprises a) at least one antibody selected in the group consisting of anti-CD14, anti-CD45, anti-CD11c, anti-CD4, anti-HLA-DR, anti-CD1c, anti-CD3, anti-CD19, anti-CD20, anti-CD56 and mixtures thereof, b) at least one antibody selected in the group consisting of anti-CD141-BD Horizon™BV711 anti-CD123-PerCP-Cy™5.5 anti-CD303-APC antibodies and mixtures thereof, and optionally anti-CD16, anti-CD86 and/or anti-CD11 b antibodies.

In a particular embodiment, the said kit for detecting at least a subset of dendritic cells selected in the group consisting of CD141+ cDC cells, CD123+ pDC cells,CD303+ pDC cells and mixtures thereof, further comprises an anti-PDL2 antibody, preferably anti-PDL2-BD Horizon™BV786.

In a particular embodiment, the kit comprises anti-CD141 and anti-PDL2 antibodies.

In a particular embodiment, the kit comprises anti-CD123 and anti-PDL2 antibodies.

In a particular embodiment, the kit comprises anti-CD303 and anti-PDL2 antibodies.

In a particular embodiment, the kit comprises anti-CD141, anti-CD123 and anti-PDL2 antibodies.

In a particular embodiment, the kit comprises anti-CD141, anti-CD303, and anti-PDL2 antibodies.

In a particular embodiment, the kit comprises anti-CD141, anti-CD123, anti-CD303 and anti-PDL2 antibodies.

In a particular and preferred embodiment, the kit of the invention comprises at least 13 antibodies comprising (a) anti-CD14, anti-CD45, anti-CD11c, anti-CD4, anti-HLA-DR, anti-CD1c, anti-CD3, anti-CD19, anti-CD20, anti-CD56 and mixtures thereof, b) anti-CD141, anti-CD123 or anti-CD303, and (c) anti-PDL2 antibodies, and optionally anti-CD16, anti-CD86 and/or anti-CD11 b antibodies.
Preferably, the said kit of the invention comprises (a) anti-CD14-BD Horizon™BV650, anti-CD45-V500, anti-CD11c-BD Horizon™BV421, anti-CD4-Alexa Fluor® 488, anti-HLA-DR-APC/ AlexaFluor 750, anti-CD1c-PE, anti-CD3-Alexa Fluor®700, anti-CD19-Alexa Fluor®700, anti-CD20-Alexa Fluor®700, anti-CD56-Alexa Fluor®700 and mixtures thereof, b) anti-CD141-BD Horizon™BV711, and/or anti-CD123-PerCP-Cy™5.5 or anti CD303-APC, and (c) anti-PDL2-BD Horizon™BV786, and optionally anti-CD16-BD Horizon™PE-CF594, anti-CD86-PE-Cy™7, anti-CD11 b-BD Horizon™BV605 and anti-CD303-APC.

Examples of commercial labelled antibodies for preparation of said kits, are referred above.

The said kit may be used also for quantifying the said 'CD markers' (cluster of differentiation marker) and so, may be used for monitoring the expression of said 'CD markers' at different points of time and/or during a therapeutic treatment to assess its therapeutic efficacy, as disclosed hereunder in the *in vitro* methods of the invention.

The kit may usually contain also at least buffers and instructions for use.
The kit may also contain 'positive controls' such as stabilized blood or standardized microbeads coated with specific antigen.

Another example of kit of the invention is a kit comprising b) at least an antibody selected in the group consisting of anti-CD141, anti-CD123 anti-CD303 and mixtures thereof, coupled to magnetic beads to purify the DC subsets and (c) pairs or primers capable of hybridizing with a part of the human PDL2 sequence (Gene: ID: 80380) or a probe capable of hybridizing with a part of the human PDL2 sequence (Gene: ID: 80380).

The invention also relates to a use of a compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and preferably additionally a compound for detecting PDL2 on said subset(s) of dendritic cells or mixtures thereof, and/or on CD1c⁺ cDC cells, or use of a combination as defined above according to the invention or a kit as defined in the invention in an *in vitro* method of diagnosing juvenile inflammatory arthritis (JIA) or septic arthritis (SA) and/or monitoring the disease progression of JIA or SA and/or assessing the efficacy of a therapy in a subject affected by JIA or SA.
Such *in vitro* methods are disclosed hereunder.

In a particular embodiment, the invention also concerns a use of compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and preferably additionally a compound for detecting PDL2 on said subset(s) of dendritic cells or mixtures thereof, and/or on CD1c⁺ cDC cells, or use of a combination as defined above according to the invention or a kit as defined in the invention for determining, in a subject affected by a juvenile arthritic form, if the juvenile arthritic form is a juvenile inflammatory arthritis (JIA) or is a septic arthritis (SA). In fact, it is a particular advantage of the invention to allow the discrimination between the both juvenile arthritic forms.

For such discrimination between JIA and SA, the said specific markers of subsets of dendritic cells as disclosed above, or combination or said kit of the invention comprises at least an antibody selected in the group consisting of anti-CD141, anti-CD123, anti-CD303 and mixtures thereof, and anti-PDL2. In a particular embodiment, the said combination of kit for discriminating between JIA and SA comprises at least 13 antibodies comprising a) anti-CD14, anti-CD45, anti-CD11c, anti-CD4, anti-HLA-DR, anti-CD1c, anti-CD3, anti-CD19, anti-CD20, anti-CD56, b) anti-CD141 , anti-CD123 or anti-CD303, and c) anti-PDL2, and optionally anti-CD16, anti-CD86 and/or anti-CD11b antibodies, and preferably labeled antibodies comprising (a) anti-CD14-BD Horizon™BV650, anti-CD45-V500, anti-CD11c-BD Horizon™BV421, anti-CD4-Alexa Fluor® 488, anti-HLA-DR-APC/ AlexaFluor 750, anti-CD1c-PE, anti-CD3-Alexa Fluor®700, anti-CD19-Alexa Fluor®700, anti-CD20-Alexa Fluor®700, anti-CD56-Alexa Fluor®700, b) anti-CD141-BD Horizon™BV711, anti-CD123-PerCP-Cy™5.5 or anti CD303-APC , and c) anti-PDL2-BD Horizon™BV786, and optionally anti-CD16-BD Horizon™PE-CF594, anti-CD86- PE-Cy™7, anti-CD11b- BD Horizon™BV605 antibodies.

### In vitro method of quantification of markers of dendritic cells

The present invention also relates to an *in vitro* method of quantifying (i) at least one subset of dendritic cells selected in the group consisting of CD141⁺ cDC cells, CD123⁺ pDC cells, CD303⁺ pDC cells, and mixtures thereof, and preferably additionally quantifying (ii) level of expression of PDL2 on said subset(s) or mixtures thereof, and/or on CD1c⁺ cDC cells, in a biological sample of a subject, in particular in a peripheral blood sample or synovial fluid or synovial tissue sample of said subject, comprising:
a) contacting the said biological sample with
   a1) a compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, preferably additionally a compound for detecting PDL2 on said subsets of dendritic cells or mixtures thereof, and/or on CD1c⁺ cDC cells, or
   a2) a combination of compounds as defined in the invention,
b) quantifying the binding of said compounds to the said biological sample.

In a particular embodiment, the present invention relates to an *in vitro* method of quantifying (i) at least one subset of dendritic cells selected in the group consisting of CD141⁺ cDC cells, CD123⁺ pDC cells CD303⁺ pDC cells and mixtures thereof, in a biological sample, in particular in a synovial fluid or synovial tissue sample of said subject, comprising:
a) contacting the said biological sample with the antibodies as defined above, in particular at least an antibody selected in the group consisting of anti-CD141 anti-CD123, anti-CD303 antibodies and mixtures thereof, preferably selected in the group consisting of anti-CD141- BD Horizon™BV711 , anti-CD123-PerCP-Cy™5.5 anti-CD303-APC antibodies and mixtures thereof;
b) quantifying the binding of said compounds to the said biological sample.

In another particular embodiment, the present invention relates to an *in vitro* method of quantifying expression level of PDL2 on said subset(s) of dendritic cells selected in the group consisting of CD141⁺ cDC cells, CD123⁺ pDC cells, CD303⁺ pDC cells and mixtures thereof, and/or CD1c⁺ cDC cells, in a biological sample, in particular in a peripheral blood sample or synovial fluid or synovial tissue sample of said subject, comprising:
a) contacting the said biological sample with the compounds for detecting PDL2 as defined above, in particular at least anti-PDL2 antibody, preferably anti-PDL2-BD Horizon™BV786 antibody;
b) quantifying the binding of said antibody to the said biological sample.

In a particular embodiment, the step a) of the *in vitro* method of quantification of said markers, is performed by contacting the said biological sample by a combination of antibodies as disclosed above. This step a) may be performed by different technologies of detection and/or selection and/or isolation (e.g purification) of subsets of cells. This step a) is also named 'gating strategy' or 'selection gating strategy'.
In a particular and preferred embodiment, this step a) is performed by flow cytometry.

As disclosed above, dendritic cells may be identified by specific antigens (e.g., HLA-DR, CD11c, CD1c, CD141 or CD123 or CD303) combined with morphometric characteristics (e.g. size, granulometry, etc.) and exclusion of contaminating cells.

Expression of these cell surface antigens may be notably assessed using well known technologies such as cell membrane staining using biotinylation or other equivalent techniques followed by immunoprecipitation with specific antibodies, flow cytometry, western blot, ELISA or ELISPOT, antibodies microarrays, or tissue microarrays coupled to immunohistochemistry. Other suitable techniques include FRET or BRET, single cell microscopic or histochemistery methods using single or multiple excitation wavelength and applying any of the adapted optical methods, such as electrochemical methods (voltametry and amperometry techniques), atomic force microscopy, and radio frequency methods, e.g. multipolar resonance spectroscopy, confocal and non-confocal, detection of fluorescence, luminescence, chemiluminescence, absorbance, reflectance, transmittance, and birefringence or refractive index (e.g., surface plasmon resonance, ellipsometry, a resonant mirror method, a grating coupler waveguide method or interferometry), cell ELISA, radioisotopic, magnetic resonance imaging, analysis by polyacrylamide gel electrophoresis (SDS-PAGE); HPLC-Mass Spectroscopy; Liquid Chromatography/Mass Spectrometry/Mass Spectrometry (LC-MS/MS)).

In a preferred embodiment, the detection of these cell surface antigens is performed by a selection gating strategy by flow cytometry and use of a combination of compounds, preferably a combination of antibodies, as disclosed above. Flow cytometry is a powerful technology that allows researchers and clinicians to perform complex cellular analysis quickly and efficiently by analyzing several parameters simultaneously. The amount of information obtained from a single sample can be further expanded by using multiple fluorescent reagents. The information gathered by the flow cytometer can be displayed as any combination of parameters chosen by the skilled person.

According to this embodiment, each of the antibodies is labelled with a specific fluorochrome as disclosed above, enabling the cytometer to identify the cells carrying the antigen recognized by said antibody.

In a preferred embodiment, the step a) requires to detect a substantially pure dendritic cells population, that is, populations of dendritic cells that are devoid of contaminant cells. As used herein, "contaminant cells" or "contaminant white blood cells" refer to the white blood cells which are present in the biologic sample of the subject and which are not dendritic cells. Such contaminant cells include granulocytes, e.g. neutrophils, eosinophils, basophils, and lymphocytes, e.g., T cells, NK cells, B cells, but also precursors of these cell types.

The remaining dendritic cells are identified and counter-selected on the basis of the expression of specific markers.

The existence of markers which are specific for each of the contaminant cell types enables the identification of these cells in the blood sample of the subject. Identified contaminant cells can then be removed from the analysis (i.e., by retaining only the data pertaining to the dendritic cells populations for the analysis), so that the study then only focuses on the dendritic cell populations. In this respect, although any of the above-mentioned analytical techniques can be used to identify the said contaminant white blood cells, flow cytometry is particularly adapted for this task, since it enables the skilled person to eliminate the contaminants and analyze the dendritic cells populations with minimal effort.

In this respect, any antibodies directed against one or more antigens expressed by one or more of the contaminant cells can be used to identify the said contaminant white blood cells. In a particular embodiment, antibodies specific for well-known antigens expressed by granulocytes (eg CD16), T lymphocytes (eg CD3), B lymphocytes (eg CD19 and CD20), and/or NK cells (eg CD56) can be used in step a).

The sequences of the clusters of differentiation disclosed above are well known in the art, and can be retrieved under the accession numbers (S76824.1 for CD16; 916 for CD3; 930 for CD19; 931 for CD20; 4684 for CD56). The sequences of these proteins are represented by the sequences AAA53507.1; NP_000724.1; NP_001761.3; NP_690605.1; NP_851996.2 respectively).

The identification of the various contaminant or cells of interest by flow cytometry can be performed sequentially or simultaneously. In a particular embodiment, the identification of the various contaminant cells in the sample is performed simultaneously using antibodies specific for CD16 to exclude macrophage and granulocytes, CD3 to exclude T cell, CD19 and CD20 to exclude B cells and CD56 to exclude NK cells. All these antibodies can be used as a single conjugated fluorochrome to detect all contaminant cells.

According to a specific embodiment, the biological sample of the subject is contacting with a combination of at least 13 antibodies as disclosed above, ie CD141, CD123 or CD303, PDL2, CD14, CD45, CD11c, CD4, HLA-DR, CD1c, CD3, CD19, CD20, and CD56, each of which recognizing a specific antigen expressed by the dendritic cells or by one or more of the contaminant cells, and each of which being labelled with a specific fluorochrome.

In a particular embodiment, the biological sample of the subject is contacting with a combination of labelled antibodies with different fluorochromes as disclosed above : PDL2-BD Horizon™BV786, CD141-BD Horizon™BV711, CD14-BD Horizon™BV650, CD45-V500, CD11c-BD Horizon™BV421, CD4-Alexa Fluor® 488, CD123-PerCP-Cy™5.5 or CD303-APC, HLA-DR-APC/ AlexaFluor 750, CD1c-PE, CD3-Alexa Fluor®700, CD19-Alexa Fluor®700, CD20-Alexa Fluor®700, CD56-Alexa Fluor®700. Additional optional antibodies may be added to this minimal combination of 13 antibodies, in particular selected in the group consisting of CD11b, CD86, CD16, being labelled with specific fluorochromes, such as CD11b-BD Horizon™BV605 antibodies, CD86-PE-Cy™7, and CD16-BD Horizon™PE-CF594. For example, CD16 which improves the elimination of granulocytes, CD303 which permits confirmation of identification of pDC double positive (CD123, CD303) and CD86 which permits the analysis of the DC activation. So the combination of antibodies used *in vitro* methods of the present invention contains at least 13 antibodies, and optionally at least 14 antibodies (with CD16), 15 antibodies (with CD16 and CD303), 16 antibodies (with CD16, CD303 and CD86) or even 17 antibodies (with CD16, CD303, CD86 and CD11b).

An optimal combination of antibodies used in the said *in vitro* method of the present invention comprises 15 antibodies with 12 specific fluorochromes: PDL2-BD Horizon™BV786, CD141-BD Horizon™BV711, CD14-BD Horizon™BV650, CD45-V500, CD11c-BD Horizon™BV421, CD4-Alexa Fluor® 488, CD123-PerCP-Cy™5.5, HLA-DR-APC/ AlexaFluor 750, CD1c-PE, CD3- Alexa Fluor®700, CD19- Alexa Fluor®700, CD20-Alexa Fluor®700, CD56- Alexa Fluor®700, CD16-BD Horizon™PE-CF594 and CD303-APC.
Examples of labelled antibodies used according to the invention are disclosed above. The sample is then analyzed by flow cytometry.

In a particular embodiment, it is advantageous to analyze only the CD141 and CD123 expressing-cells, in order to eliminate the contaminant cells and to select cells, including all the dendritic cells. In this embodiment, cells were plotted according to their size and granulometry followed by doublets and dead cells exclusions. First CD45 positive/SSC intermediate population is selected and contaminant cells were excluded using several markers (CD3, CD19, CD20, CD56). CD4 positive cells with high expression of HLA-DR were subsequently gated to delineate two major subsets of DC, the myeloid subsets (CD11c⁺⁺HLA-DR⁺⁺CD123^{low}) and the plasmacytoid subsets or CD123 pDCs (CD11c^{low}HLA-DR⁺⁺CD123^{high}). Within myeloid cells, CD141 positive cells delineate CD141⁺ cDC and CD1c positive cells delineate the CD1c⁺ cDC subset. InflDC are defined as CD14 positive cells within CD1c⁺ cDC. Numeration of cells was performed by addition of known numbers of fluorescent beads (Flow-Count™ Fluorosphere, Beckman Coulter) before acquisition of cells which allow the calculation of absolute numbers of cells for each cell subsets.

Expression of cell surface CD141 and CD123 (or CD303) on dendritic cells may be assessed using specific antibodies, in particular using well known technologies such as cell membrane staining using biotinylation (or other equivalent techniques), followed by immunoprecipitation with specific antibodies, flow cytometry, western blot, ELISA or ELISPOT, antibodies microarrays, or tissue microarrays coupled to immunohistochemistry.

Preferably, the expression of cell surface protein selected in the group consisting of CD141, CD123, CD303 and mixtures thereof, is detected by flow cytometry. Flow cytometry is a useful tool for simultaneously measuring multiple physical properties of individual particles (such as cells). Cells pass single-file through a laser beam. As each cell passes through the laser beam, the cytometer records how the cell or particle scatters incident laser light and emits fluorescence. Using a flow cytometric analysis protocol, one can perform a simultaneous analysis of surface molecules at the single-cell level.

In this embodiment, the use of fluorochromic agents attached to anti-CD141, anti-CD123 and anti-CD303 antibodies to enable the flow cytometer to detect on the basis of size, granularity and fluorescent light is highly advantageous. Thus, the flow cytometer can be configured to provide information about the relative size (forward scatter or "FSC"), granularity or internal complexity (side scatter or "SSC"), and relative fluorescent intensity of the cell sample. The fluorescent light detects on the basis of CD141-expressing or CD123-expressing or CD303-expressing, enabling the cytometer to identify these dendritic cells.

It is necessary to use a combination of antibodies as disclosed above at the same time, ie at least a) anti-CD14, anti-CD45, anti-CD11c, anti-CD4, anti-CD3, anti-CD19, anti-CD20, anti-CD56, anti-HLA-DR antibodies, anti-CD1c antibodies, b) anti-CD141 and/or anti-CD123 and/or anti-CD303, and preferably additionally c) anti-PDL2 antibody, and optionally anti-CD86, and/or anti-CD16 antibodies. These antibodies are labelled with various fluorophores emitting in distinguishable wavelengths. This strategy enables the identification of 3 types of dendritic cells with respect to CD1c, CD141 and CD123 expression and the expression of inhibitory/activating molecules at the cell surface (eg PDL2 and CD86).

In another particular embodiment of the invention, step a) comprises a step of isolating dendritic cells by cell enrichment using commercial isolation kits, such as the ones disclosed hereunder:
1-DC isolation, using either the Pan-DC Enrichment Kit (Ref 130-100-777 from Miltenyi Biotech), or Blood DC isolation kit (Ref 130-091-379 from Miltenyi Biotech) that allows the concurrent isolation of untouched pDC, CD1c+ cDC, and cCD141 cDC by depletion of non-DCs, i.e., T cells, B cells, NK cells, monocytes, granulocytes, and erythroid cells using a cocktail of MicroBead-conjugated antibodies against antigens that are not expressed by DCs;
2-positive selection of pDC in one step procedure using a kit (Ref 130-097-149 from Miltenyi Biotech), or two steps procedure (Ref 130-097-140 from Miltenyi Biotech) using a cocktail of biotin-conjugated antibodies against lineage-specific antigens to pre-enriched pDCs which are labeled with CD304 (BDCA-4/Neuropilin-1) MicroBeads;
3-positive selection of both CD1c+ and CD141+ myeloid DC, using a myeloid DC isolation kit (Ref 130-094-487 from Miltenyi Biotech).

Following DC enrichment, analyses of PDL2 expression in DC subsets could be analyzed by quantitative PCR, Western Blot, ELISA or others equivalent technologies.

### In vitro method of diagnosing JIA or SA and in vitro method of discriminating between these both arthritic forms

Another object of the invention is an *in vitro* method of diagnosing juvenile inflammatory arthritis (JIA) in a subject, said method comprising the steps of:
a) quantifying according to the invention and as disclosed above, in a biological sample of a subject, in particular in a synovial fluid or synovial tissue sample of said subject, at least one subset of dendritic cells selected in the group consisting of CD141⁺ cDC cells, CD123⁺ pDC cells, CD303⁺ pDC cells, and mixtures thereof,
b) comparing said values of subset(s) of dendritic cells of step a) with a reference value,
wherein an increase of the value of said subset(s) of dendritic cells in comparison to the reference value is indicative of a subject affected by juvenile inflammatory arthritis (JIA).

The invention also concerns an *in vitro* method of diagnosing septic arthritis (SA) in a subject, said method comprising the steps of:
a) quantifying according to the invention and as disclosed above, in a biological sample of a subject, in particular a peripheral blood sample or synovial fluid or synovial tissue sample of said subject, the expression level of PDL2 on at least one subset of dendritic cells selected in the group consisting of CD141⁺cDC cells, CD123⁺pDCs cells, CD303⁺ pDCs cells and mixtures thereof, and/or CD1c⁺ cDC cells,
b) comparing the expression level of PDL2 on said subset(s) of cells of step a) with a reference value,
wherein an increase of the expression level of PDL2 on said subset(s) of dendritic cells in comparison to a reference value is indicative of a subject affected by septic arthritis (SA).

Advantageously, another object of the invention concerns an *in vitro* method of discriminating, in a subject affected by an arthritic form, if the arthritic form is a juvenile inflammatory arthritis (JIA) or a septic arthritis (SA), said method comprising the steps of:
a) implementing the *in vitro* method of diagnosing JIA according to the invention, and in case of no increase of the value of subset(s) of dendritic cells selected in the group consisting of CD141⁺ cDC cells, CD123⁺ pDC cells, CD303⁺ pDC cells, and mixtures thereof, in comparison to the reference value, indicative of a subject unaffected by JIA,
b) implementing the *in vitro* method of diagnosing SA according to the invention to determine if the subject is affected by SA.

In a particular embodiment, the step a) in the *in vitro* methods of diagnosing JIA or SA as disclosed above, is performed by cell membrane staining using biotinylation (or other equivalent techniques), followed by immunoprecipitation with specific antibodies, flow cytometry, western blot, ELISA or ELISPOT, antibodies microarrays, or tissue microarrays coupled to immunohistochemistry, preferably flow cytometry.

In a particular and preferred embodiment, the step a) is performed by flow cytometry.

The diagnosis *in vitro* methods of the invention can be practiced with any antibody or antiserum detecting (or recognizing specifically) the antigens expressed by the dendritic cells. In a particular embodiment, the combination of antibodies as disclosed above, in particular the combination with at least 13 antibodies as disclosed above is used.

The present inventors have surprisingly found that the numbers of subsets of dendritic cells selected in the group consisting of CD141⁺ cDC cells, CD123⁺ pDC cells, CD303⁺ pDC cells, and mixtures thereof, in synovial fluid sample of a subject is sufficient to discriminate JIA compared to SA. They therefore propose to use the numbers of subsets of dendritic cells selected in the group consisting of CD141⁺ cDC cells, CD123⁺ pDC cells, CD303⁺ pDC cells, and mixtures thereof, in synovial fluid sample of a subject as a positive diagnosis criterion for JIA.

The number of subsets of dendritic cells selected in the group consisting of CD141⁺ cDC cells, CD123⁺ pDC cells, CD303⁺ pDC cells, and mixtures thereof, in synovial fluid sample of a subject is compared with a reference value to determine if the said subject suffers from JIA.

The term "reference value", as used herein, refers to the expression level of a JIA diagnosis marker under consideration (e.g. CD141⁺ cDC and/or CD123⁺ pDC and/or CD303⁺ pDC in synovial fluid sample) in a reference sample. A "reference sample", as used herein, means a biological sample obtained from a subject under the same conditions as the "tested sample" to which it is compared with; for example, both "tested" and "reference" samples are synovial fluid obtained under the same conditions.

In a first embodiment, the "reference sample" for a JIA "tested sample" is a biological sample coming from a SA subject.

In another embodiment, the "reference sample" for a JIA "tested sample" is a biological sample coming from a "control subject" as defined above.

In another embodiment, the "reference sample" for a JIA "tested sample" or SA "tested sample" is a biological sample coming from the same subject being tested (diagnosed with JIA or respectively SA) but at a different point of time, preferably at an earlier point of time, ie a time 'T' before the time of the "tested sample" named "T+ x", wherein "x" is expressed in days, months or years, said comparison being useful to monitor the progress of the pathology.

In the examples as illustrated hereunder, the "reference sample" for a JIA "tested sample" is a "biological sample" coming from SA subject.

The reference value or reference level can be an absolute value; a relative value; a value that has an upper or a lower limit; a range of values; an average value; a median value, a mean value, or a value as compared to a particular control or baseline value. A reference value can be based on an individual sample value. The reference value can be based on a large number of samples, such as from population of subjects of the chronological age matched group, or based on a pool of samples including or excluding the sample to be tested.

In a particular embodiment, the reference values for the number of CD141 cDC, CD123 pDC or the CD303 pDC in the biological fluid are absolute numbers calculated using Flow-Count Fluorospheres (Beckman coulter); a suspension of fluorescent microbeads may be used to determine absolute counts on the flow cytometer. In contrast, the reference values for the expression of PDL2 are relative values that may depend on antibody conjugates, instrument performance, variations in instrument settings, and differences in log amplifiers or digital signal processing systems that can invalidate comparisons of fluorescent measurement, between instruments and between laboratories. An alternative to this semi-quantitative measures is to perform quantitative flow cytometry, using measurement of the mean number of fluorescently labeled antibodies bound per cell (ABC). This can be accomplished using standard beads to construct calibrating curves for comparison to a cellular specimen. Quantum™ Simply Cellular® (QSC) (Bangs Laboratories) and QuantiBRITE (QuantiBRITE standard beads and QuantiCALC software; BD Biosciences, San Jose, CA) or QUANTUM™ MESF assays are designed to quantitate the binding of antibodies directly conjugated to fluorochromes.

In the QSC assay, standard beads coated with antimouse immunoglobulin with precalibrated antibody binding capacities are incubated with the antibody being studied.

In the QuantiBRITE or QUANTUM™ MESF (Molecule of soluble fluorochrome) assay, standard beads with varying amount of PE molecules for Quantibrite or Alexa Fluor® 488, Alexa Fluor® 647, APC, Cy™5, PE-Cy™5, R-PE, Pacific Blue™, and FITC for QUANTUM™ MESF are used to generate a calibration curve. Using this curve, ABC values for various-labeled antibodies can be determined in cell populations of interest. If the Fluorochrome to antibody ratio is 1:1, then the number of fluorescently labeled antibodies bound per cell (from the calibration curve) equals the number of antibody molecules bound per cell. When the assay is performed under conditions of saturation binding, ABC values give a measure of the overall surface expression of the antigen.

Previous studies indicate that ABC determinations using the QuantiBRITE assay are simple and consistent and that QuantiBRITE PE beads can be employed as a tool for standardized analysis across instruments and laboratories. Other studies demonstrated that QSC beads with known antibody-binding capacity enable determination of absolute numbers of cellular epitopes and can also be used for standardization across channels with different sensitivities

The quantification of subsets of dendritic cells selected in the group consisting of CD141⁺ cDC cells, CD123⁺ pDC cells, CD303⁺ pDC cells, and mixtures thereof thus preferably involves as disclosed above, contacting the subject's biological sample with an antibody selected in the group consisting of an anti-CD141 antibody , an anti-CD123 antibody, an anti-CD303 antibody and mixtures thereof, so as to determinate the level of surface CD141 expression and/or CD123 expression and/or CD303 expression.

The PDL2 expression level on said subsets of dendritic cells selected in the group consisting of CD141⁺ cDC cells, CD123⁺ pDC cells, CD303⁺ pDC cells, and mixtures thereof and/or on CD1c⁺ cDC cells, in blood and/or in synovial fluid sample of a subject permits to discriminate between SA and JIA, the subject affected by SA having an increase level expression of PDL2.

The quantification of PDL2 expression level on said subsets of dendritic cells selected in the group consisting of CD141⁺ cDC cells, CD123⁺ pDC cells, CD303⁺ pDC cells, and mixtures thereof and/or on CD1c⁺ cDC cells, preferably involves contacting the subject's biological sample with an anti-PDL2 antibody so as to determinate the level of PDL2 expression. In an alternative embodiment, the quantification of PDL2 expression is performed by the use of a pair of primers capable of hybridizing at least a part of human PDL2 sequence (Gene: ID: 80380) or a probe capable of hybridizing at least a part of human PDL2 sequence (Gene: ID: 80380).

### In vitro method of monitoring the disease progression

The present invention also concerns an *in vitro* method for monitoring the disease progression in a subject affected by JIA comprising the steps of:
a) quantifying according to the invention and at a first point of time, in a biological sample of a subject, in particular in a synovial fluid or synovial tissue sample of said subject, first values of subset(s) of dendritic cells selected in the group consisting of CD141⁺cDC, CD123⁺ pDCs, CD303⁺ pDCs, and mixtures thereof,
b) quantifying according to the invention and at a second point of time, in a biological sample of a subject, in particular in a synovial fluid or synovial tissue sample of said subject, second values of subset(s) of dendritic cells selected in the group consisting of CD141⁺cDC, CD123⁺ pDCs, CD303⁺ pDCs, and mixtures thereof,,
c) comparing the first and second values obtained at step a) and step b), and
d) monitoring the disease progression in the subject affected by JIA from the said comparison.

The invention also relates to an in vitro method for monitoring the disease progression in a subject affected by SA comprising the steps of:
a) quantifying according to the invention and at a first point of time, in a biological sample of a subject, in particular in a peripheral blood sample or synovial fluid or synovial tissue sample of said subject, first values for expression level of PDL2 on subset(s) of dendritic cells selected in the group consisting of CD141⁺cDC, CD123⁺ pDCs, CD303⁺ pDCs, and mixtures thereof, and/or on CD1c⁺ cDC cells,
b) quantifying according to the invention and at a second point of time, in a biological sample of the same subject, in particular in a peripheral blood sample or a synovial fluid or synovial tissue sample of said subject, second values for expression level of PDL2 on subset(s) of dendritic cells selected in the group consisting of CD141⁺cDC, CD123⁺ pDCs, CD303⁺ pDCs, and mixtures thereof, and/or on CD1c⁺ cDC cells,
c) comparing the first and second values obtained at step a) and step b), and
d) monitoring the disease progression in the subject affected by SA from the said comparison.

### In vitro method for assessing the efficacy of a therapy

The invention also relates to an *in vitro* method for assessing the efficacy of a therapy in a subject affected by JIA, comprising the steps of:
a) quantifying according to the invention as disclosed above, in a biological sample of a subject before said treatment, in particular in a synovial fluid or synovial tissue sample of said subject, first values of subset(s) of dendritic cells selected in the group consisting of CD141⁺cDC, CD123⁺ pDCs, CD303⁺ pDCs, and mixtures thereof,,
b) quantifying according to the invention as disclosed above, in a biological sample of the same subject during or after said treatment, in particular in a synovial fluid or synovial tissue sample of said subject, first values of subset(s) of dendritic cells selected in the group consisting of CD141⁺cDC, CD123⁺ pDCs, CD303⁺ pDCs, and mixtures thereof,
c) assessing the efficacy of the therapy based on the comparison of the second values obtained in step b) with the first values obtained at step a).

Examples of therapy assessed for JIA are Non-steroidal anti-inflammatory drugs (NSAIDs) and Disease-modifying anti rheumatic dugs (DMARDs) (e.g methotrexate) as well as Steroids and biological therapies (e.g etanercept, adalimumab, tocilizumab).

Another object of the invention is an *in vitro* method for assessing the efficacy of a therapy in a subject affected by SA, comprising the steps of:
a) quantifying according to the invention as disclosed above, in a biological sample of a subject before said treatment, in particular in a peripheral blood sample or a synovial fluid or synovial tissue sample of said subject, first values for expression level of PDL2 on subset(s) of dendritic cells selected in the group consisting of CD141⁺cDC, CD123⁺ pDCs, CD303⁺ pDCs, and mixtures thereof, and/or on CD1c⁺ cDC cells,
b) quantifying according to the invention as disclosed above, in a biological sample of the same subject during or after said treatment, in particular in a peripheral blood sample or a synovial fluid or synovial tissue sample of said subject, first values for expression level of PDL2 on subset(s) of dendritic cells selected in the group consisting of CD141⁺cDC, CD123⁺ pDCs, CD303⁺ pDCs, and mixtures thereof,, and/or on CD1c⁺ cDC cells,
c) assessing the efficacy of the therapy based on the comparison of the second values obtained in step b) with the first values obtained at step a).

Examples of therapy assessed for SA repeated joint aspirations with washing were preferentially performed and arthrotomy was performed if necessary associated with intravenous antibiotics adapted to local epidemiology (e.g : cefazoline).

### Use of markers

The present invention also relates to a use of at least one cell surface protein selected in the group consisting of CD141, CD123, CD303, and mixtures thereof as specific markers of dendritic cells for diagnosing JIA and/or monitoring the disease progression JIA and/or assessing the efficacy of a therapy in a subject affected by juvenile inflammatory arthritis (JIA) from biological sample of a subject, in particular a synovial fluid or synovial tissue sample of a subject.

The present invention also concerns use of PDL2 as a specific marker of dendritic cells for diagnosing SA and/or monitoring the disease progression SA and/or assessing the efficacy of a therapy in a subject affected by septic arthritis (SA) from a peripheral blood sample or a synovial fluid or synovial tissue sample of a subject.

The present invention will be now illustrated in the non-limitative examples.

### EXAMPLES

### Example 1: Identification of markers of SA and JIA by flow cytometric immunophenotyping

### 1.1 Materials and methods

### Patients

All the 8 JIA included in this study fulfilled the ILAR classification criteria for JIA and were prospectively recruited from the Pediatric Department at the CHU in Montpellier. Patients from 6 months to 15 years having acute arthritis requiring joint puncture were recruited. Seven out of the 8 JIA were oligoarthritis and 1 enthesis-related. Children with intra-articular puncture contraindication (platelet under 50 000/mm3, reduced prothrombine, prolonged activated partial thromboplastin time), JIA patient treated by biotherapies, corticoids or other immunosuppressors in the month preceding the articular draining, SA patients treated by antibiotic that started more than 24h before draining time were excluded from the study. The SA patients included were all confirmed either bacteriologically after SF or hemoculture or by PCR on SF using *Kingella kingae* specific- and 16S rDNA specific primers for amplification of the bacterial DNA. Ethics committees namely CPP (Comité de Protection des Personnes sud méditerranée I) approval have been obtained (2014-A01561-46).

These samples were collected after informed consent according to the Declaration of Helsinki.

### Laboratory assays

The analysis of antibodies, erythrocyte sedimentation rate, C-reactive protein (CRP) as well as medical records have been evaluated and obtained as part of the routine diagnosis evaluation of JIA at the Department of Immunology (CHU Montpellier).

### Reagents

All the following antibodies are commercialized references.

The V450-conjugated anti-CD3, Alexa Fluor®700-conjugated anti CD3, BV650-conjugated anti-CD4, FITC-conjugated anti-CD4, BV605-conjugated anti-CD11 b, BV421-conjugated anti-CD11c, BV650-conjugated anti-CD14, PE-Cy™7-conjugated anti-CD14, Alexa Fluor®700-conjugated anti-CD16, BV421-conjugated anti-CD16, BD Horizon™PE-CF594-conjugated anti-CD16, phycoerythrin (PE)-conjugated anti-CD19, Alexa Fluor®700-conjugated anti-CD19, Alexa Fluor®700-conjugated anti-CD20, V500-conjugated anti-CD45, BV786-conjugated anti-NKp46, Alexa Fluor®700-conjugated anti-CD56, PE-conjugated anti-CD64, PE-Cy™7-conjugated anti-CD86, PerCP-Cy™5.5-conjugated anti-CD123, BD Horizon™BV711-conjugated anti-CD141, BD Horizon™BV786-conjugated anti-PDL2, and BD Horizon™PE-CF594-conjugated anti-CD163 were from BD Biosciences. APC-Cy7 anti-HLA-DR was purchased from Beckman Coulter. PE-conjugated anti CD1c, APC-conjugated anti-CD303 and APC-conjugated anti-Slan antibodies were purchased from Miltenyi Biotech. FITC-conjugated anti-CD192 antibody was purchased from R&D Systems. All antibodies were used according to their manufacturer recommendations.

### Flow cytometric immunophenotyping

Granulocytes, T and B lymphocytes as well as NK staining was performed on 50µl fresh whole heparin-treated blood using CD4, CD45, CD3, CD19, CD16, CD56 markers. DC staining (PDL2, CD141, CD14, CD45, CD11c, CD4, CD123 or alternatively CD303 or CD304, CD3, CD19, CD20, CD56, HLA-DR, CD1c, and optionally CD86, CD16, and CD11b) and monocyte staining (NKp46, CD11b, CD45, CD16, CD14, CD163, CD64, HLA-DR, CD3, CD19, CD56, CD192, SLAN) was performed on 300 µl of fresh whole heparin-treated blood. After labelling, gentle lysis of erythrocytes was performed with addition of EasyLyse™ solution (Dako).

Synovial cellular fractions were recovered following centrifugation (300g, 10min) of heparin-treated SF. One million of cells were stained using the same antibodies panels than the whole blood after Fc block.

For monocytes staining, debris and doublets were excluded using SSC/FSC staining followed by selection of DAPI negative CD45⁺ viable leucocytes. Contaminating granulocytes (HLA-DR^{neg}CD16^{high}) and lineage positive cells (CD3, CD19 and CD56) were excluded from monocytic gated cell. The three populations of monocytes were gated within HLA-DR positive cells. The percentage of the classical monocytes (CD14⁺⁺CD16⁻), intermediate monocytes (CD14⁺CD16⁺) and non-classical monocytes (CD14⁺CD16⁺⁺) are given within the gated monocytes following exclusion of double negative cells.

For the DC staining, cells were plotted according to their size and granulometry followed by doublets and dead cells exclusions. First CD45 positive/SSC intermediate population is selected and contaminant cells were excluded using lineage markers (CD3, CD19, CD20, CD56). CD4 positive cells with high expression of HLA-DR were subsequently gated to delineate two major subsets of DC, the myeloid subsets (CD11c⁺⁺HLA-DR⁺⁺CD123^{low}) and the plasmacytoid subsets or CD123 pDCs (CD11c^{low}HLA-DR⁺⁺CD123^{high}). Alternatively, CD303 is used instead of CD123 for detecting the myeloid subsets (CD11c⁺⁺HLA-DR⁺⁺CD303^{low}) and the plasmacytoid subsets or CD303 pDCs (CD11c^{low}HLA-DR⁺⁺CD303^{high}). CD304 may also be used instead of CD303. But in a particular and preferred embodiment, CD123 is used. Within myeloid cells, CD141 positive cells delineate CD141⁺ cDC and CD1c positive cells delineate the CD1c⁺ cDC subset. InflDC are defined as CD14 positive cells within CD1c⁺ cDC. Numeration of cells was performed by addition of known numbers of fluorescent beads (Flow-Count™ Fluorosphere, Beckman Coulter) before acquisition of cells which allow the calculation of absolute numbers of cells for each cell subsets. All data were acquired using BD-LSR Fortessa (BD Biosciences). Compensation analyses were performed using DIVA software and all data were analyzed using FlowJo software (Tree Star, Ashland, OR, USA).

### Hierarchical clustering

Analyses were performed using Perseus software (Tyanova S et al., Nat Methods. 2016;13(9):731-740). Cell counts and expression markers (mean fluorescence intensity) were plotted in volcano plot to determine differences, a false discovery rate (FDR) cutoff of 5% was used to build the heatmaps.

### Statistics

For each DC subset, data are presented as mean ± SEM of total cell count per millilitre of biological fluid. Statistical significance between the two disease groups (JIA and SA) was determined using Student t test or Mann Withney according to the distribution of the data. Analyses were performed with a bilateral alpha level of 0.05 using SAS software, version 9.4 (SAS Institute, Cary, NC, USA).

Blood pDC numbers and disease duration correlation was analyzed by Spearman Rank correlation. P values of less than 0.05 (*P <0.05), ** P<0.01, *** P<0.001 were considered significant.

### 1.2 Differences in monocytes and DC subsets counts discriminate JIA and SA patients

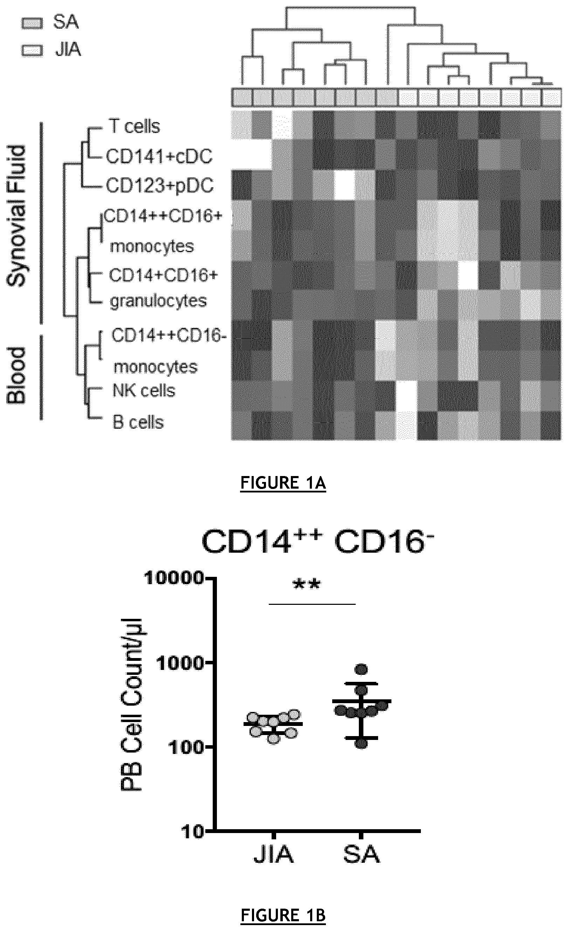

To evaluate whether immune cell types could better discriminate between JIA and SA patients, an in-depth immunophenotyping of monocytes and dendritic cells was performed using multiparametric flow cytometry. Based on the CD14 and CD16 phenotypic markers, the three main monocyte subsets were defined: classical, intermediate and non-classical monocytes in peripheral blood (PB) and synovial fluid (SF). While the three monocyte subsets were found in PB, the non-classical monocyte subset was barely detected in SF. Interestingly, in both biologic fluids, the total count of monocytes was significantly increased in SA compared with JIA. Indeed, in PB, monocytes counts were 231 ± 16 in JIA versus 431 ± 95 in SA (p=0.0027) and in SF, 225 ± 69 in JIA versus 3187 ±1689 (p<0.0001). CD1c⁺ and CD141⁺ conventional (c)DC cell counts were analyzed in both fluids, as well as CD123⁺ DCs (pDCs) and CD14⁺ inflDCs (inflammatory DCs). Unsupervised hierarchical clustering was applied to cytometric data, i.e. cell counts of all immune cell types analyzed in PB and SF. This unbiased clustering approach showed two major clades of samples, which stratified the patients into JIA and SA, and encouraged to further analyze the data sets. Using a false discovery rate (FDR) cutoff of 5%, the cell counts that were significantly differentially expressed were analyzed. Heat map showed a clear separation between sepsis and inflammatory juvenile arthritis using 7 SF and 4 PB parameters, indicating an overall pattern of disease-related changes mainly within joints (Figure 1A). The majority of the immune cell subsets that discriminate both diseases (8 out of 11) are higher in SA than in JIA. Indeed, among all parameters tested, only total T cells, CD123⁺ pDCs and CD141⁺ cDCs numbers were higher in JIA SF than in SA. Overall, these data suggest that monocytes and granulocytes are the main immune cells over-represented in SA while in JIA T cells and DCs are predominant.

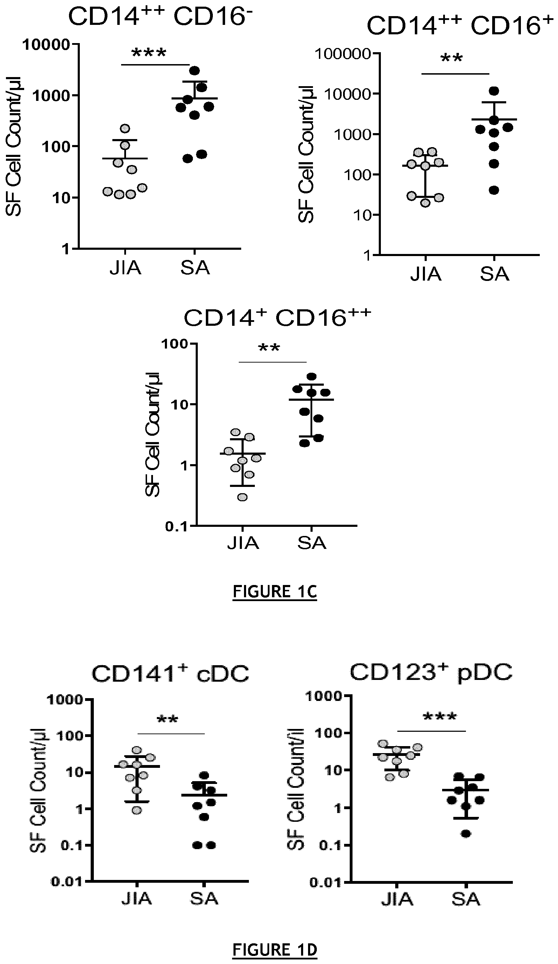

Significant differences in cell counts in monocyte subsets were subsequently examined. In PB, only classical monocyte counts were significantly higher in SA compared with JIA (Figure 1B). In the SF, the numbers of the classical, intermediate and non-classical monocyte subsets were significantly higher in SA than JIA (Figure 1C). These data evidenced a differential accumulation of monocyte subsets in the periphery and joints of SA patients.

Furthermore, CD141⁺ cDCs were easily detected in the SF isolated from JIA patients, and in significantly higher numbers than in SA. Numbers of CD123⁺ DCs (pDCs) were also significantly higher in the SF of JIA than in SA. Altogether these data demonstrated a significant higher CD141⁺ cDCs and CD123⁺ DCs (pDCs) cell counts in the SF of JIA patients compared with SA. (Figure 1D).

### 1.3 Specific activation markers on monocyte and DC subsets discriminate both diseases

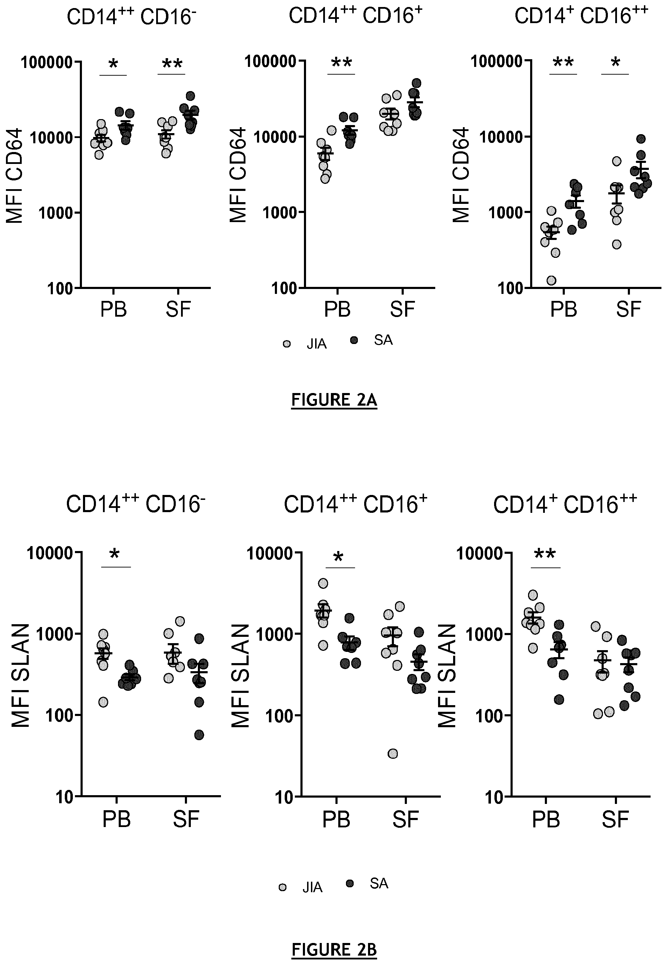

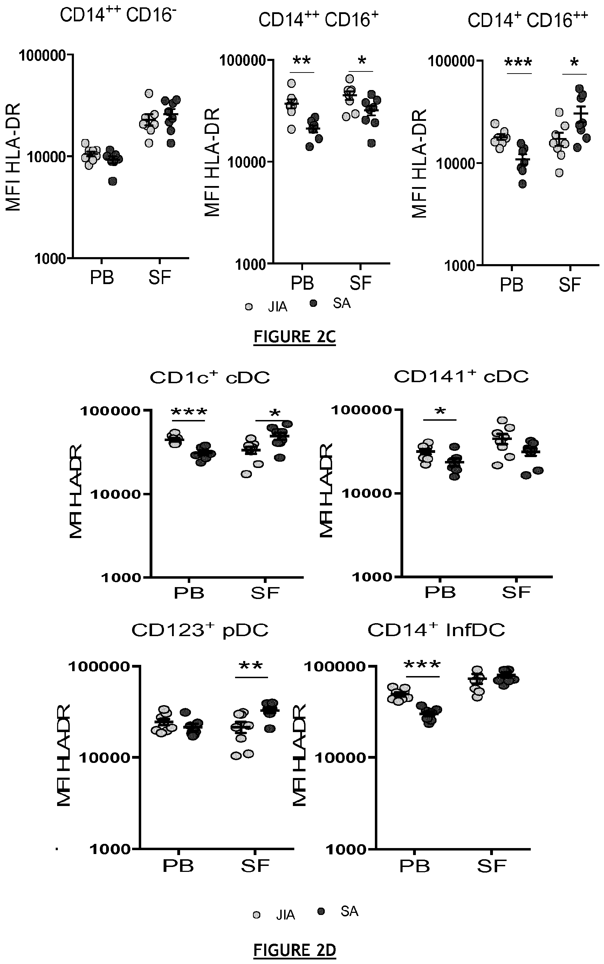

Since monocytes and DCs are crucial in regulating T cell responses against foreign and self-antigens, their activation status in the SF and PB of JIA and SA was further analyzed. The expression levels of co-stimulatory HLA-DR and CD86 molecules, as well as the inhibitory PDL2 receptor were monitored on four DC subsets. The expression levels of HLA-DR, CD163, SLAN and CD64 markers as well as CCR2 were monitored on the three monocytes subsets. The expression of CD64 was higher in SA than in JIA for the three monocyte subsets in both fluids (Figure 2A). On the contrary, SLAN expression was markedly increased on the three monocytes subsets of JIA compared with SA, but only significantly in the PB (Figure 2B). The expression level of HLA-DR was significantly higher on intermediate CD14⁺⁺CD16⁺ monocytes isolated from both PB and SF and on non-classical monocytes CD14⁺CD16⁺⁺ from PB in JIA than in SA patients. Conversely, HLA-DR expression was higher on non-classical CD14⁺CD16⁺⁺ monocytes of the SF of SA patients than JIA patients (Figure 2C). Overall, HLA-DR, SLAN and CD64 expressions on monocyte subsets discriminate between JIA and SA with a peculiar focus on the CD64 expression on classical monocytes from the SF that could discriminate both arthritis.

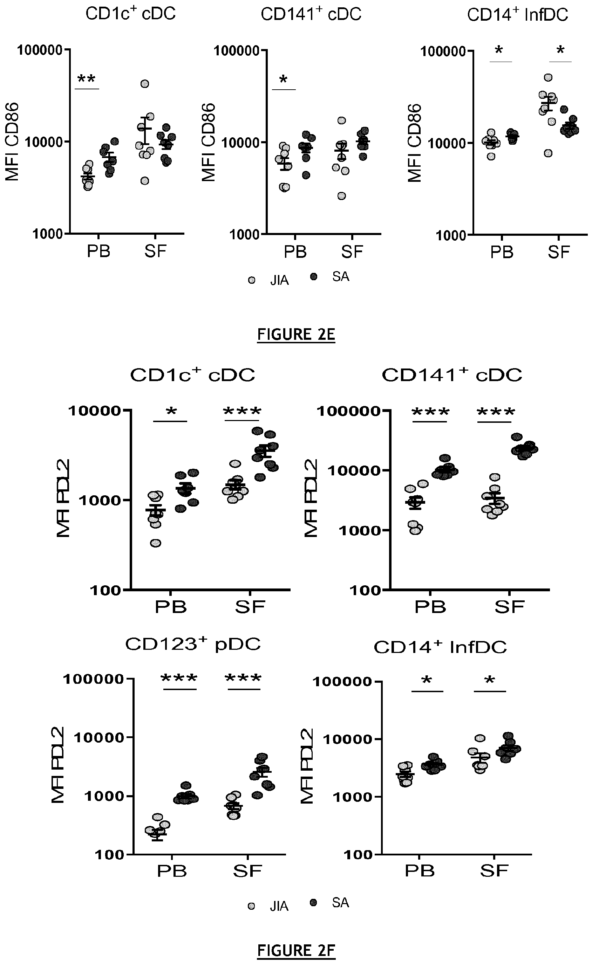

HLA-DR expression levels were significantly elevated on CD1c⁺ and CD141⁺ cDCs, as well as on CD14⁺ InflDCs in blood JIA whereas HLA-DR expression levels were significantly higher on CD1c⁺ cDC and CD123⁺pDC from SA SF compared with JIA (Figure 2D). CD86 expression was significantly increased on CD141⁺ cDCs and CD14⁺InfDC of SA PB and conversely on CD14⁺ InfDCs of JIA SF compared with SA (Figure 2E). Strikingly, the expression level of PDL2 was significantly elevated in all four DC subsets isolated from both the SF and PB of SA patients compared to JIA patients (Figure 2F). Overall, these data suggest that activation markers of DC subsets in both fluids are increased, while the inhibitory PDL2 molecule is decreased, in JIA patients compared with SA patients.

### 1.4 PDL2 expression on DC subsets discriminates between JIA and SA patients

For each maturation marker, Receiver Operating Characteristic (ROC) analyses based on the maximal Youden index were performed in order to define a cut-off point that would help identifying the probability to discriminate between JIA and SA (data not shown). The area under the ROC curve (AUC) provided an assessment of the performance of the test. For all maturation markers, a cut-off point was selected at 0.95. All p values were less than 0.0001 and markers that were not correlated with age were selected. Based on these criteria, 8 markers that showed great potential to discriminate between JIA and SA were identified. In the PB, were selected HLA-DR expression on CD1c⁺ cDCs and CD14⁺ InfDC, as well as CD86 expression on CD141+ cDC and PDL2 expression on CD141⁺ cDCs and CD123⁺ pDCs. The potential predictors in the SF were PDL2 expression on CD1c⁺, CD141⁺ cDCs and CD123⁺ pDCs (Figure 2F). These data suggest that the monitoring of PDL2 expression on blood or SF DCs may be a convenient and rapid biomarker to discriminate both diseases at diagnosis.

In summary, infectious and autoimmune diseases have often been associated with altered monocytes or DC numbers, indicating a pathogenic role of certain subsets in these pathologies. Overall, the present study showed that both monocytes and DC numbers might be of interest to discriminate SA and JIA, mainly when monitored in SF, with a particular interest in CD123⁺ pDC numbers. When considering activation markers, our data suggest that both peripheral blood (PB) and synovial fluid (SF) could be monitored. Potential predictors discriminating both diseases might be HLA-DR expression on CD1c⁺ cDCs and CD14⁺ InfDC, as well as PDL2 expression on CD141⁺ cDCs and CD123⁺ pDCs, in the PB; and PDL2 expression on CD1c⁺ and CD141⁺ cDCs, as well as on CD123⁺ pDCs in the SF. In conclusion, our study contributes to the identification of new markers discriminating both diseases that may help clinical decision.

### Example 2: Use of selected markers for discriminating SA and JIA subjects

DC cells were obtained from fresh synovial fluid (SF) as disclosed above. Synovial cellular fractions were recovered following centrifugation (300g, 10min) of heparin-treated SF. One million of cells were stained using the same antibodies panels than the whole blood after Fc block.

Subsets of DC cells from SF were selected and analyzed using flow cytometry and level of expression of PDL2 was also analyzed, as disclosed above.

The phenotypic characterization of DC cells was performed on a fresh sample (unfrozen) within 6 hours following the articular puncture. The sample contained at least 0.5 million of cells.

The 17 antibodies used in the present example were coupled to 14 different fluorochromes : PDL2-BD Horizon™BV786 (Ref : 563843, BD Biosciences), CD141-BD Horizon™BV711 (Ref 563155, BD Biosciences), CD14-BD Horizon™BV650 (Ref : 563419, BD Biosciences), CD11b- BD Horizon™BV605 (Ref: 562721, BD Biosciences), CD45-V500 (Ref 560777, BD Biosciences), CD11c-BD Horizon™BV421 (Ref 562561, BD Biosciences), CD4-Alexa Fluor® 488 (Ref 557695, BD Biosciences), CD123- PerCP-Cy™5.5 (Ref 558714, BD Biosciences), CD86-PE-CyTM7 (Ref 561128, BD Biosciences), CD16-BD Horizon™PE-CF594 (Ref 562293, BD Biosciences), CD1c-PE (Ref 130-090-508, Miltenyi Biotech), HLA-DR-APC/Alexa Fluor®750 (Ref B42021, Beckman Coulter), CD3-Alexa Fluor®700 (Ref 557943, BD Biosciences), CD19- Alexa Fluor®700 (557921, BD Biosciences), CD20- Alexa Fluor®700 (Ref 560631, BD Biosciences), CD56- Alexa Fluor®700 (Ref 557919, BD Biosciences). All antibodies were used according to their manufacturer recommendations.

The gating strategy was the same as disclosed above in example 1 to select subsets CD141⁺ cDC and CD123+ pDCs.

Cells were plotted according to their size and granulometry followed by doublets and dead cells exclusions. First CD45 positive/SSC intermediate population is selected and contaminant cells were excluded using several markers (CD3, CD19, CD20, CD56). CD4 positive cells with high expression of HLA-DR were subsequently gated to delineate two major subsets of DC, the myeloid subsets (CD11c⁺⁺HLA-DR⁺⁺CD123^{low}) and the plasmacytoid subsets or CD123 pDCs (CD11c^{low}HLA-DR⁺⁺CD123^{high}). Within myeloid cells, CD141 positive cells delineate CD141⁺ cDC and CD1c positive cells delineate the CD1c⁺ cDC subset. InflDC are defined as CD14 positive cells within CD1c⁺ cDC. Numeration of cells was performed by addition of known numbers of fluorescent beads (Flow-Count™ Fluorosphere, Beckman Coulter) before acquisition of cells which allow the calculation of absolute numbers of cells for each cell subsets. All data were acquired using BD-LSR Fortessa (BD Biosciences). Compensation analyses were performed using DIVA software and all data were analysed using FlowJo software (Tree Star, Ashland, OR, USA). All the cell count (Number of cells / µl of fluid) and MFI data presented are Mean ± SEM.

Hierarchical clustering analyses were performed using Perseus software (Tyanova S et al., Nat Methods. 2016;13(9):731-740). Cell counts and semi-quantitative expression markers (mean fluorescence intensity) were plotted in volcano plot to determine differences, a false discovery rate (FDR) cutoff of 5% was used to build the heatmaps.

These data altogether showed that an accumulation of CD141⁺ cDC and/or CD123⁺ pDCs cells in synovial fluid (SF) is indicative of JIA diagnosis and an increased expression of PDL2 on DC cells from PB or SF, in particular on CD1c, CD141⁺ cDC and/or CD123⁺ pDCs of SF, is indicative of SA diagnosis.

References values can thus be determined for the diagnosis of JIA or SA. These references values are given into the following Table 1. The reference values for the expression of PDL2 are relative values (MFI) that are determined for each populations of interest and can be compared between patients. The reference values for the number of CD141⁺ cDC, CD123⁺ pDC or the CD303⁺ pDC in the synovial fluid are absolute numbers calculated using Flow-Count Fluorospheres (Beckman coulter).

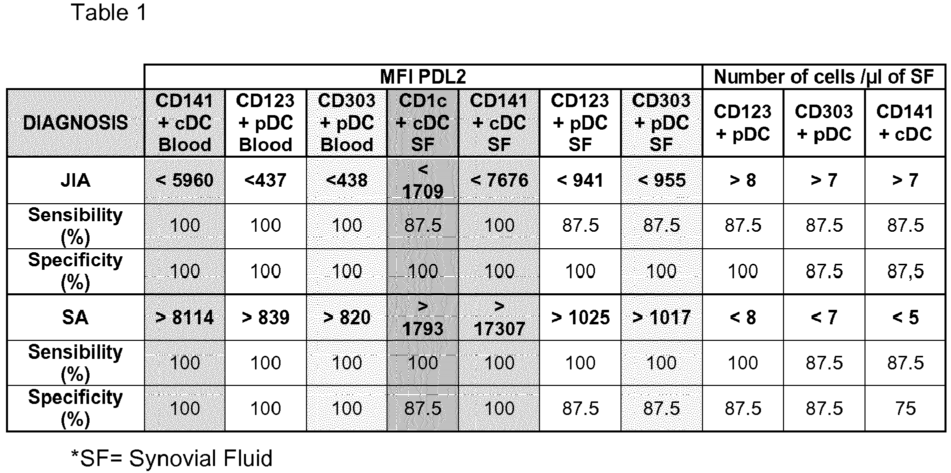

Sensitivity (also called the true positive rate) measures the proportion of actual positives that are correctly identified as such (e.g., the percentage of JIA subject who are correctly identified as having the condition).

Specificity (also called the true negative rate) measures the proportion of actual negatives that are correctly identified as such (e.g., the percentage of non-JIA subject who are correctly identified as not having the condition JIA).

These specific markers and antibodies produced to specifically detect the presence and/or level of such markers may be used in *in vitro* methods of diagnosis and/or monitoring the progression of SA or JIA in a subject.

Combinations and kits comprising specific antibodies designed to bind these DC markers and reagents may be produced accordingly.

### 1.5 Monitoring disease duration and assessing the efficacy of therapy

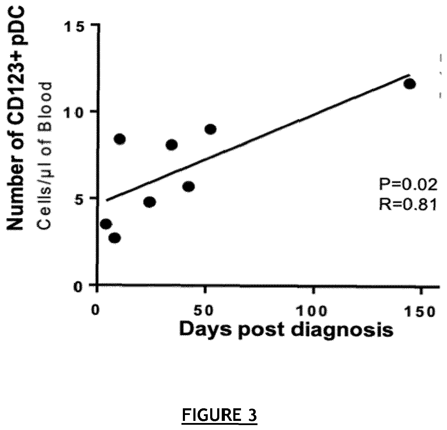

Variation in blood pDC numbers was found significantly correlated with disease duration with a positive correlation (Figure 3). This result suggests that the monitoring of blood pDC numbers may help to assess the efficacy of the treatment in JIA patient.

## Claims

1. A combination which comprises:
a) at least one compound for detecting a cell surface protein selected in the group consisting of CD14, CD45, CD11c, CD4, HLA-DR, CD1c, CD3, CD19, CD20, and CD56 and mixtures thereof, and optionally CD16, CD86 and/or CD11b, and
b) at least one compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and
c) preferably additionally a compound for detecting PDL2 on subsets of dendritic cells selected in the group consisting of CD141⁺cDC, CD123⁺pDCs, CD303⁺ pDCs cells and mixtures thereof, and/or CD1c⁺ cDC cells,.

2. The combination of claim 1, wherein:
a) the compound(s) for detecting a cell surface protein selected in the group consisting of CD14, CD45, CD11c, CD4, HLA-DR, CD1c, CD3, CD19, CD20, and/or CD56 and mixtures thereof, and optionally CD16, CD86, and/or CD11b, is (are) anti-CD14, anti-CD45, anti-CD11c, anti-CD4, anti-HLA-DR, anti-CD1c, anti-CD3, anti-CD19, anti-CD20, anti-CD56, and optionally anti-CD16, anti-CD86 and/or anti-CD11 b antibodies,
b) the compound (i) for detecting CD141⁺ cDC cells is an anti-CD141 antibody, the compound (ii) for detecting CD123⁺ pDC cells is an anti-CD123 antibody and the compound (iii) for detecting CD303⁺ pDC is an anti-CD303 antibody, and
c) the compound for detecting PDL2 is selected in the group consisting of (x) an anti-PDL2 antibody, (y) a pairs of primers capable of hybridizing at least a part of human PDL2 sequence (Gene: ID: 80380) or (z) a probe capable of hybridizing at least a part of human PDL2 sequence (Gene: ID: 80380).

3. The combination according to claim 2, wherein it comprises at least 13 antibodies comprising a) anti-CD14, anti-CD45, anti-CD11c, anti-CD4, anti-HLA-DR, anti-CD1c, anti-CD3, anti-CD19, anti-CD20, anti-CD56 antibodies, b) anti-CD141 antibody, anti-CD123 or anti-CD303 antibodies, and (c) anti-PDL2 antibody, and optionally anti-CD16, anti-CD86 and/or anti-CD11 b antibodies.

4. The combination according to claim 2 or claim 3, wherein the said antibodies are labelled with a detectable marker, in particular selected in the group consisting of enzymes, prosthetic groups, fluorescent materials, luminescent materials, bioluminescent materials, and radioactive materials, preferably fluorescent materials, in particular the said labelled antibodies are selected in the group consisting of a) anti-CD14- BD Horizon™BV650, anti-CD45-V500, anti-CD11c-BD Horizon™BV421, anti-CD4- Alexa Fluor® 488, anti-HLA-DR-APC/ AlexaFluor 750, anti-CD1c-PE, anti-CD3- Alexa Fluor®700, anti-CD19-Alexa Fluor®700, anti-CD20-Alexa Fluor®700, anti-CD56-Alexa Fluor®700, b) anti-CD141-BD Horizon™BV711, anti-CD123- PerCP-Cy™5.5 or anti-CD303-APC and c) anti-PDL2- BD Horizon™BV786, and optionally anti-CD16-BD Horizon™PE-CF594, anti-CD86-PE-Cy™7, and/or anti-CD11 b- BD Horizon™BV605 antibodies.

5. A kit comprising at least a combination as defined in anyone of claims 1 to 4.

6. Use of a compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and preferably additionally a compound for detecting PDL2 on said subsets of dendritic cells or mixtures thereof and/or on CD1c⁺ cDC cells, or use of a combination according to anyone of claims 1 to 4 or a kit of claim 5 for determining, in a subject affected by a juvenile arthritic form, if the juvenile arthritic form is a juvenile inflammatory arthritis (JIA) or is a septic arthritis (SA).

7. An *in vitro* method of quantifying (i) at least one subset of dendritic cells selected in the group consisting of CD141⁺ cDC cells, CD123⁺ pDC cells, CD303⁺ pDC cells and mixtures thereof, and preferably additionally quantifying (ii) expression level of PDL2 on said subset(s) of dendritic cells or mixtures thereof, and/or on CD1c⁺ cDC cells, in a biological sample of a subject, in particular in a peripheral blood sample or a synovial fluid or synovial tissue sample of said subject, comprising:
a) contacting the said biological sample with
a1) a compound selected in the group consisting of (i) a compound for detecting CD141⁺ conventional dendritic cells (cDC), (ii) a compound for detecting CD123⁺ plasmacytoid dendritic cells (pDC), (iii) a compound for detecting CD303⁺ plasmacytoid dendritic cells (pDC), and mixtures thereof, and preferably additionally a compound for detecting PDL2 on said subsets of dendritic cells or mixtures thereof, and/or on CD1c⁺ cDC cells, or
a2) a combination of compounds as defined in anyone of claims 1 to 4,
b) quantifying the binding of said compounds to the said biological sample.

8. An *in vitro* method of diagnosing juvenile inflammatory arthritis (JIA) in a subject, said method comprising the steps of:
a) quantifying, according to claim 7, in a biological sample of a subject, in particular in a synovial fluid or synovial tissue sample of said subject, at least one subset of dendritic cells selected in the group consisting of CD141⁺ cDC cells, CD123⁺ pDC cells, CD303⁺ pDC cells, and mixtures thereof,
b) comparing said values of said subset(s) of dendritic cells of step a) with a reference value,
wherein an increase of the value of said subset(s) of dendritic cells in comparison to the reference value is indicative of a subject affected by juvenile inflammatory arthritis (JIA).

9. An *in vitro* method of diagnosing septic arthritis (SA) in a subject, said method comprising the steps of:
a) quantifying, according to claim 7, in a biological sample of a subject, in particular a peripheral blood sample or synovial fluid or synovial tissue sample of said subject, the expression level of PDL2 on at least one subset of dendritic cells selected in the group consisting of CD141⁺cDC cells, CD123⁺pDCs cells, CD303⁺ pDCs cells, and mixtures thereof, and/or CD1c⁺ cDC cells,
b) comparing the expression level of PDL2 on said subset(s) of dendritic cells of step a) with a reference value,
wherein an increase of the expression level of PDL2 on said subset(s) of dendritic cells in comparison to a reference value is indicative of a subject affected by septic arthritis (SA).

10. The method of claim 8 or claim 9, wherein the step a) is performed by cell membrane staining using biotinylation (or other equivalent techniques), followed by immunoprecipitation with specific antibodies, flow cytometry, western blot, ELISA or ELISPOT, antibodies microarrays, or tissue microarrays coupled to immunohistochemistry, preferably flow cytometry.

11. An *in vitro* method of discriminating, in a subject affected by an arthritic form, if the arthritic form is a juvenile inflammatory arthritis (JIA) or a septic arthritis (SA), said method comprising the steps of:
a) implementing the *in vitro* method of diagnosing JIA according to claim 8, and in case of no increase of the value of subset(s) of dendritic cells selected in the group consisting of CD141⁺ cDC cells, CD123⁺ pDC cells, CD303⁺ pDC cells and mixtures thereof in comparison to the reference value, indicative of a subject unaffected by JIA,
b) implementing the *in vitro* method of diagnosing SA according to claim 9 to determine if the subject is affected by SA.

12. An *in vitro* method for monitoring the disease progression in a subject affected by JIA comprising the steps of:
a) quantifying at a first point of time, according to claim 7, in a biological sample of a subject, in particular in a synovial fluid or synovial tissue sample of said subject, first values of subset(s) of dendritic cells selected in the group consisting of CD141⁺cDC cells, CD123⁺ pDCs cells, CD303⁺ pDCs cells and mixtures thereof,
b) quantifying at a second point of time, according to claim 7, in a biological sample of a subject, in particular in a synovial fluid or synovial tissue sample of said subject, second values of subset(s) of dendritic cells selected in the group consisting of CD141⁺cDC cells, CD123⁺ pDCs cells, CD303⁺ pDCs cells and mixtures thereof,
c) comparing the first and second values obtained at step a) and step b), and
d) monitoring the disease progression in the subject affected by JIA from the said comparison.

13. An *in vitro* method for monitoring the disease progression in a subject affected by SA comprising the steps of:
a) quantifying at a first point of time, according to claim 7, in a biological sample of a subject, in particular in a peripheral blood sample or a synovial fluid or synovial tissue sample of said subject, first values for expression level of PDL2 on subsets of dendritic cells selected in the group consisting of CD141⁺cDC cells, CD123⁺ pDCs cells, CD303⁺ pDCs cells, CD1c⁺ cDC cells and mixtures thereof,
b) quantifying at a second point of time, according to claim 7, in a biological sample of the same subject, in particular in a peripheral blood sample or a synovial fluid or synovial tissue sample of said subject, second values for expression level of PDL2 on subset(s) of dendritic cells selected in the group consisting of CD141⁺cDC cells, CD123⁺ pDCs cells, CD303⁺ pDCs cells, and mixtures thereof, and/or CD1c⁺ cDC cells,
c) comparing the first and second values obtained at step a) and step b), and
d) monitoring the disease progression in the subject affected by SA from the said comparison.

14. An *in vitro* method for assessing the efficacy of a therapy in a subject affected by JIA, comprising the steps of:
a) quantifying, according to claim 7, in a biological sample of a subject before said treatment, in particular in a synovial fluid or synovial tissue sample of said subject, first values of subset(s) of dendritic cells selected in the group consisting of CD141⁺cDC cells, CD123⁺ pDCs cells, CD303⁺ pDCs, and mixtures thereof,
b) quantifying, according to claim 7, in a biological sample of the same subject during or after said treatment, in particular in a synovial fluid or synovial tissue sample of said subject, first values of subset(s) of dendritic cells selected in the group consisting of CD141⁺cDC cells, CD123⁺ pDCs cells , CD303⁺ pDCs cells, and mixtures thereof,
c) assessing the efficacy of the therapy based on the comparison of the second values obtained in step b) with the first values obtained at step a).

15. An *in vitro* method for assessing the efficacy of a therapy in a subject affected by SA, comprising the steps of:
a) quantifying, according to claim 7, in a biological sample of a subject before said treatment, in particular in a peripheral blood sample or a synovial fluid or synovial tissue sample of said subject, first values for expression level of PDL2 on subset(s) of dendritic cells selected in the group consisting of CD141⁺cDC cells, CD123⁺ pDCs cells, CD303⁺ pDCs cells and mixtures thereof, and/or CD1c⁺ cDC cells,
b) quantifying, according to claim 7, in a biological sample of the same subject during or after said treatment, in particular in a peripheral blood sample or a synovial fluid or synovial tissue sample of said subject, first values for expression level of PDL2 on subset(s) of dendritic cells selected in the group consisting of CD141⁺cDC cells, CD123⁺ pDCs cells, CD303⁺ pDCs cells and mixtures thereof, and/or CD1c⁺ cDC cells,
c) assessing the efficacy of the therapy based on the comparison of the second values obtained in step b) with the first values obtained at step a).
